(19) Europäisches Patentamt
European Patent Office
Office européen des brevets

(11) Veröffentlichungsnummer: 0 313 908 A2

(12) # EUROPÄISCHE PATENTANMELDUNG

(21) Anmeldenummer: 88116808.2

(22) Anmeldetag: 11.10.88

(51) Int. Cl.4: C07C 59/68 , C07C 69/734 , C07C 103/178

(30) Priorität: 24.10.87 DE 3736089

(43) Veröffentlichungstag der Anmeldung: 03.05.89 Patentblatt 89/18

(84) Benannte Vertragsstaaten: BE CH DE ES FR GB IT LI NL

(71) Anmelder: BAYER AG
Konzernverwaltung RP Patentabteilung
D-5090 Leverkusen 1 Bayerwerk(DE)

(72) Erfinder: Kirsten, Rolf, Dr.
Carl-Langhans-Strasse 27
D-4019 Monheim(DE)
Erfinder: Marhold, Albrecht, Dr.
Carl-Duisberg-Strasse 329
D-5090 Leverkusen 1(DE)
Erfinder: Santel, Hans-Joachim, Dr.
Gruenstrasse 9 a
D-5090 Leverkusen 1(DE)
Erfinder: Schmidt, Robert R., Dr.
Im Waldwinkel 110
D-5060 Bergisch-Gladbach 2(DE)
Erfinder: Lürssen, Klaus, Dr.
August-Kierspel-Strasse 145
D-5060 Bergisch-Gladbach 2(DE)
Erfinder: Strang, Harry, Dr.
Unterdorfstrasse 6 a
D-4000 Düsseldorf 31(DE)

(54) **Halogensubstituierte Phenoxyphenylpropionsäure-Derivate.**

(57) Die Erfindung betrifft neue halogensubstituierte Phenoxyphenylpropionsäure-Derivate der Formel

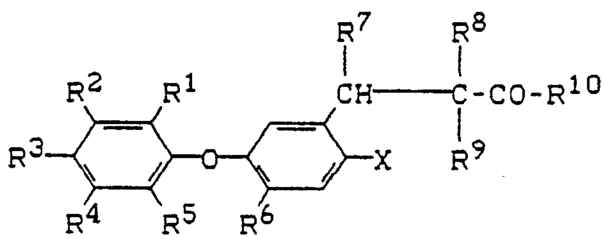

(I)

wobei die Substituenten die in der Beschreibung angegebene Bedeutung haben, mehrere Verfahren und neue Zwischenprodukte zu ihrer Herstellung sowie ihre Verwendung als Herbizide und als Pflanzenwuchsregulatoren.

EP 0 313 908 A2

## Halogensubstituierte Phenoxyphenylpropionsäure-Derivate

Die Erfindung betrifft neue halogensubstituierte Phenoxyphenylpropionsäure-Derivate, Verfahren und neue Zwischenprodukte zu ihrer Herstellung sowie ihre Verwendung als Herbizide und als Pflanzenwuchsregulatoren.

Es ist bereits bekannt, daß bestimmte Phenoxybenzoesäure-Derivate, wie z. B. 3-(2,4-Dichlor-phenoxy)-6-nitrobenzoesäure-methylester (Bifenox) herbizid wirksam sind (vgl. US-P 3 652 645 und US-P 3 776 715). Die Wirkung dieser bekannten Verbindungen ist jedoch nicht immer zufriedenstellend.

Es wurden nun neue halogensubstituierte Phenoxyphenylpropionsäure-Derivate der allgemeinen Formel (I)

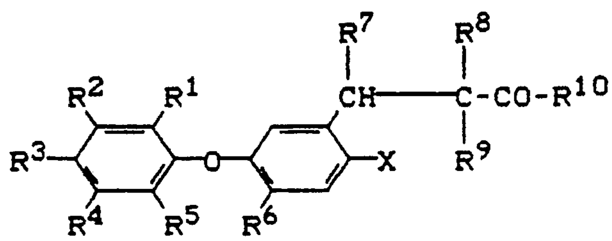

(I)

in welcher

X für Halogen steht,

$R^1$ für Wasserstoff, Halogen, Cyano oder Trifluormethyl steht,

$R^2$ für Wasserstoff oder Halogen steht,

$R^3$ für Halogen, Cyano, Trifluormethyl, Trifluormethoxy oder Trifluormethylsulfonyl steht,

$R^4$, $R^5$, $R^6$, $R^7$, $R^8$ und $R^9$ unabhängig voneinander für Wasserstoff oder Halogen stehen,

$R^{10}$ für Halogen, Hydroxy, Amino, Alkylamino, Alkenylamino, Alkinylamino, Arylamino, Aralkylamino, Alkoxycarbonyl-alkylamino, Cyanoamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonyl-alkylthio oder für die Gruppierung O-$R^{11}$ steht, worin

$R^{11}$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Trialkylsilylalkyl, Arylthioalkyl, Aralk oxyalkyl, Aralkylthioalkyl, Alkoxycarbonylalkyl, Alkylaminocarbonylalkyl, Aralkyl, Azolylalkyl oder für ein Ammonium-, Alkylammonium-, Alkalimetall- oder Erdalkalimetall-äquivalent steht oder für die Gruppierung

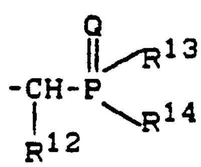

steht, worin

$R^{12}$ für Wasserstoff, Alkyl, Aryl, Furyl, Thienyl oder Pyridyl steht,

$R^{13}$ für Alkyl oder Alkoxy steht,

$R^{14}$ für Alkoxy steht und

Q für Sauerstoff oder Schwefel steht, oder

$R^{11}$ für die Gruppierung $-(CH_2)_n-R^{15}$ steht, worin

n für die Zahlen 0, 1 oder 2 steht und

$R^{15}$ für einen gegebenenfalls durch Halogen und/oder Alkyl substituierten heterocyclischen Rest aus der Reihe Furyl, Tetra hydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht,

gefunden.

Weiter wurde gefunden, daß man die neuen halogensubstituierten Phenoxyphenylpropionsäure-Derivate

der allgemeinen Formel (I) erhält, wenn man

(a) Phenoxyphenylaminoverbindungen der allgemeinen Formel (II)

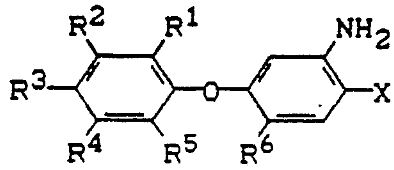

(II)

in welcher

$R^1$, $R^2$, $R^3$, $R^4$, $R^5$, $R^6$ und X die oben angegebenen Bedeutungen haben,

- oder Säureaddukte von Verbindungen der Formel (II) -

mit Natriumnitrit oder Kaliumnitrit und mit einem Hydrogenhalogenid (H-X') in Gegenwart von Wasser und gegebenenfalls in Gegenwart eines organischen Lösungsmittels umsetzt und die dabei gebildeten Diazoniumsalze der allgemeinen Formel (III)

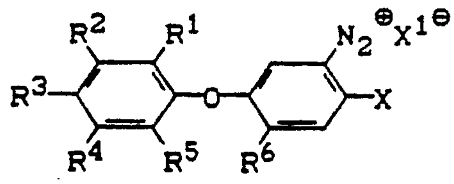

(III)

in welcher

$R^1$, $R^2$, $R^3$, $R^4$, $R^5$ und $R^6$ die oben angegebenen Bedeutungen haben und

X und $X^1$ gleich oder verschieden sind und für Halogen stehen,

mit Acrylsäure-Derivaten der allgemeinen Formel (IV)

$$H_2C=\overset{R^8}{\overset{|}{C}}-CO-R^{10} \qquad (IV)$$

in welcher

$R^8$ und $R^{10}$ die oben angegebenen Bedeutungen haben,

in Gegenwart von Hydrogenhalogeniden (HX'), gegebenenfalls in Gegenwart von Katalysatoren und gegebenenfalls in Gegenwart von Wasser und dem gegebenenfalls bei der Erzeugung der Verbindung der Formel (III) verwendeten organischen Lösungsmittel umsetzt und gegebenenfalls an den so erhaltenen Verbindungen der Formel (I) weitere Derivatisierungen in dem durch die obige Substituentendefinition vorgegebenen Rahmen nach üblichen Methoden durchführt, oder

(b) für den Fall, daß $R^7$, $R^8$ und $R^9$ für Wasserstoff stehen und die Reste $R^1$ bis $R^6$ sowie $R^{10}$ und X die oben angegebenen Bedeutungen haben, wenn man Verbindungen der Formel (I), in welcher $R^7$, $R^8$ und/oder $R^9$ für Halogen stehen und die Reste $R^1$ bis $R^6$ sowie $R^{10}$ und X die oben angegebenen Bedeutungen haben,

mit Wasserstoff in Gegenwart eines Hydrierungskatalysators und in Gegenwart eines Verdünnungsmittels umsetzt, oder

(c) ebenfalls für den Fall, daß $R^7$, $R^8$ und $R^9$ für Wasserstoff stehen und die Reste $R^1$ bis $R^6$ sowie $R^{10}$ und X die oben angegebenen Bedeutungen haben, wenn man Verbindungen der Formel (V)

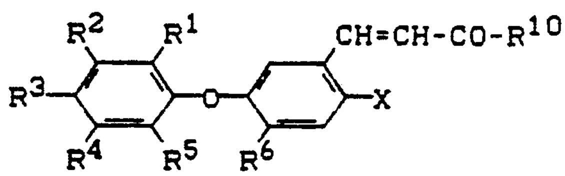

(V)

in welcher

$R^1$, $R^2$, $R^3$, $R^4$, $R^5$, $R^6$, $R^{10}$ und X die oben angegebenen Bedeutungen haben,

mit Wasserstoff in Gegenwart eines Hydrierungskatalysators und in Gegenwart eines Verdünnungsmittels umsetzt, oder

(d) für den Fall, daß $R^7$ für Chlor oder Brom steht und die Reste $R^1$ bis $R^6$ sowie $R^8$ bis $R^{10}$ und X die oben angegebenen Bedeutungen haben, wenn man Verbindungen der Formel (I), in welcher $R^7$ für Wasserstoff steht und die Reste $R^1$ bis $R^6$ sowie $R^8$ bis $R^{10}$ und X die oben angegebenen Bedeutungen haben,
mit N-Chlor- bzw. N-Brom-succinimid in Gegenwart von 2,2′-Azoisobuttersäure-dinitril (Azoisobutyronitril) und von Tetrachlormethan umsetzt, oder

(e) für den Fall, daß $R^{10}$ für Hydroxy steht und die Reste $R^1$ bis $R^9$ und X die oben angegebenen Bedeutungen haben, wenn man Verbindungen der Formel (VI)

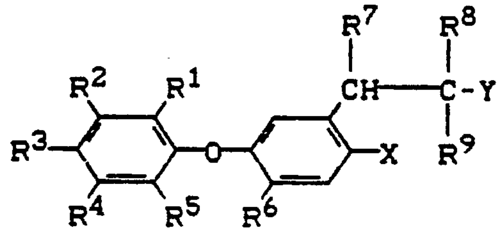

(VI)

in welcher
$R^1$, $R^2$, $R^3$, $R^4$, $R^5$, $R^6$, $R^7$, $R^8$, $R^9$ und X die oben angegebenen Bedeutungen haben und
Y für Cyano, Methoxycarbonyl oder Ethoxycarbonyl steht,
mit Wasser, gegebenenfalls in Gegenwart eines Verseifungshilfsmittels und gegebenenfalls in Gegenwart eines organischen Lösungsmittels umsetzt, oder

(f) für den Fall, daß $R^{10}$ für Halogen steht und die Reste $R^1$ bis $R^9$ und X die oben angegebenen Bedeutungen haben, wenn man Verbindungen der Formel (I), in welcher $R^{10}$ für Hydroxy steht und die Reste $R^1$ bis $R^9$ und X die oben angegebenen Bedeutungen haben,
mit einem Halogenierungsmittel, gegebenenfalls in Gegenwart eines Katalysators und gegebenenfalls in Gegenwart eines organischen Lösungsmittels, umsetzt, oder

(g) für den Fall, daß $R^{10}$ mit Ausnahme von Halogen die oben angegebene Bedeutung hat und die Reste $R^1$ bis $R^9$ und X die oben angegebenen Bedeutungen haben, wenn man Verbindungen der Formel (I), in welcher $R^{10}$ für Halogen steht und die Reste $R^1$ bis $R^9$ und X die oben angegebenen Bedeutungen haben
mit Verbindungen der Formel (VII)

$H-R^{10}$ (VII)

in welcher
$R^{10}$ mit Ausnahme von Halogen die oben angegebene Bedeutung hat,
gegebenenfalls in Gegenwart eines Säureakzeptors und gegebenenfalls in Gegenwart eines Verdünnungsmittels umsetzt, oder

(h) für den Fall, daß X für Chlor oder Brom steht und die Reste $R^1$ bis $R^{10}$ die oben angegebenen Bedeutungen haben, wenn man Verbindungen der Formel (VIII)

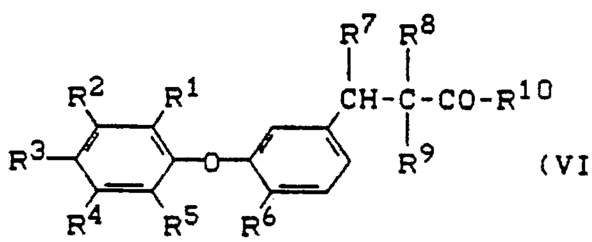

(VIII)

in welcher
$R^1$, $R^2$, $R^3$, $R^4$, $R^5$, $R^6$, $R^7$, $R^8$, $R^9$ und $R^{10}$ die oben angegebenen Bedeutungen haben,
mit Chlor oder Brom, gegebenenfalls in Gegenwart eines Verdünnungsmittels umsetzt.

Schließlich wurde gefunden, daß die neuen halogensubstituierten Phenoxyphenylpropionsäure-Derivate der Formel (I) hervorragende herbizide bzw. pflanzenwuchsregulierende Eigenschaften aufweisen.

Überraschenderweise sind die erfindungsgemäßen halogensubstituierten Phenoxyphenylpropionsäure-Derivate der Formel (I) gegen Unkräuter wesentlich stärker wirksam als 3-(2,4-Dichlor-phenoxy)-6-nitro-benzoesäure-methylester, welches ein strukturell ähnlicher vorbekannter Wirkstoff gleicher Wirkungsrichtung ist.

Die Erfindung betrifft vorzugsweise Verbindungen der Formel (I), in welcher
X für Fluor, Chlor oder Brom steht,
$R^1$ für Wasserstoff, Fluor, Chlor, Brom, Cyano oder Trifluormethyl steht,
$R^2$ für Wasserstoff, Fluor, Chlor oder Brom steht,
$R^3$ für Fluor, Chlor, Brom, Cyano, Trifluormethyl, Trifluormethoxy oder Trifluormethylsulfonyl steht,
$R^4$, $R^5$, $R^6$, $R^8$ und $R^9$ unabhängig voneinander für Wasserstoff, Fluor, Chlor oder Brom stehen,
$R^7$ für Wasserstoff, Chlor oder Brom steht,
$R^{10}$ für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxy-carbonyl-$C_1$-$C_2$-alkylamino, Cyanoamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung O-$R^{11}$ steht, worin
$R^{11}$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl oder für ein Ammonium-, ein $C_1$-$C_4$-Alkylammonium-, ein Natrium-, Kalium-oder Calcium-äquivalent steht, oder für die Gruppierung

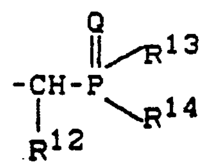

steht, worin
$R^{12}$ für Wasserstoff, $C_1$-$C_4$-Alkyl, Phenyl, Furyl, Thienyl oder Pyridyl steht,
$R^{13}$ für $C_1$-$C_4$-Alkyl oder $C_1$-$C_4$-Alkoxy steht,
$R^{14}$ für $C_1$-$C_4$-Alkoxy steht und
Q für Sauerstoff oder Schwefel steht, oder
$R^{11}$ für die Gruppierung -$(CH_2)_n$-$R^{15}$ steht, worin
n für die Zahlen 0, 1 oder 2 steht und
$R^{15}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

Die Erfindung betrifft insbesondere Verbindungen der Formel (I), in welcher
X für Chlor oder Brom steht,
$R^1$ für Wasserstoff, Fluor oder Chlor steht,
$R^2$ für Wasserstoff, Fluor oder Chlor steht,
$R^3$ für Trifluormethyl oder Trifluormethylsulfonyl steht,
$R^4$ für Wasserstoff, Fluor oder Chlor steht,
$R^5$ für Wasserstoff, Fluor oder Chlor steht,
$R^6$ für Wasserstoff oder Chlor steht,
$R^7$ für Wasserstoff, Chlor oder Brom steht,
$R^8$ für Wasserstoff, Chlor oder Brom steht,
$R^9$ für Wasserstoff oder Chlor steht,
$R^{10}$ für Chlor, Hydroxy, Amino, $C_1$-$C_4$-Alkylamino, $C_1$-$C_4$-Alkoxy-carbonyl-$C_1$-$C_2$-alkylamino, Di-($C_1$-$C_3$-alkyl)amino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Phenylsulfonylhydrazino, $C_1$-$C_4$-Alkylthio oder $C_1$-$C_4$-

Alkoxycarbonyl-$C_1$-$C_2$-alkylthio oder für die Gruppierung O-$R^{11}$ steht, worin
$R^{11}$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkyl-sulfi nyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl oder für ein Ammonium-, ein $C_1$-$C_3$-Alkylammonium-, ein Natrium- oder Kalium-äquivalent steht, oder für die Gruppierung

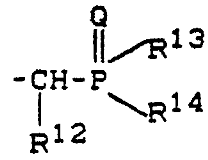

steht, worin
$R^{12}$ für Wasserstoff, Methyl, Phenyl, Furyl, Thienyl oder Pyridyl steht,
$R^{13}$ für $C_1$-$C_2$-Alkoxy steht,
$R^{14}$ für $C_1$-$C_2$-Alkoxy steht und
Q für Sauerstoff oder Schwefel steht, oder
$R^{11}$ für die Gruppierung $-(CH_2)_n-R^{15}$ steht, worin
n für die Zahlen 0, 1 oder 2 steht und
$R^{15}$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl und Dioxolanyl steht.

Verwendet man für das erfindungsgemäße Verfahren (a) 2-Chlor-5-(2-chlor-4-trifluormethyl-phenoxy)-anilin, Natriumnitrit und Salzsäure sowie anschließend Acrylsäuremethylester als Ausgangsstoffe, so kann der Reaktionsablauf durch das folgende Formelschema wiedergegeben werden:

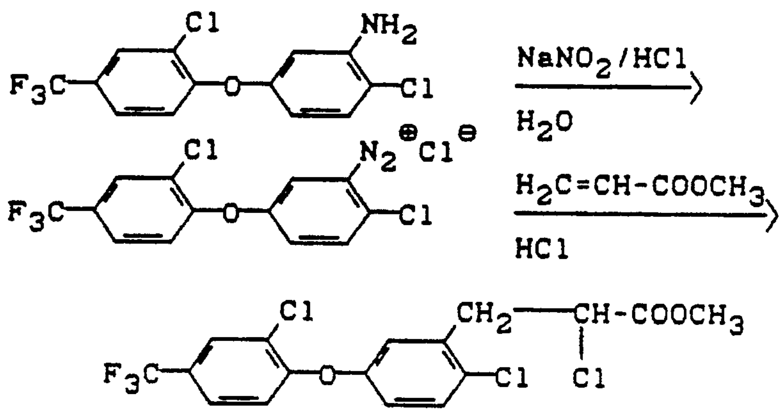


Verwendet man für das erfindungsgemäße Verfahren (b) beispielsweise 2-Chlor-3-(2-chlor-5-(2-chlor-6-fluor-4-trifluormethyl-phenoxy)-phenyl)-propionsäureethylester als Ausgangsstoff und Raney-Nickel als Katalysator, so kann der Reaktionsablauf durch das folgende Formelschema wiedergegeben werden:

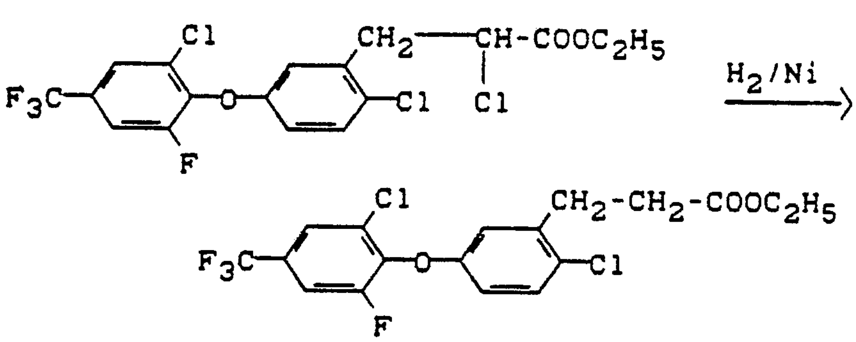


Verwendet man für das erfindungsgemäße Verfahren (c) beispielsweise 3-(2-Chlor-5-(2,6-dichlor-4-trifluormethylphenoxy)-phenyl)-acrylsäuremethylester als Ausgangsstoff und Raney-Nickel als Katalysator,

so kann der Reaktionsablauf durch das folgende Formelschema skizziert werden:

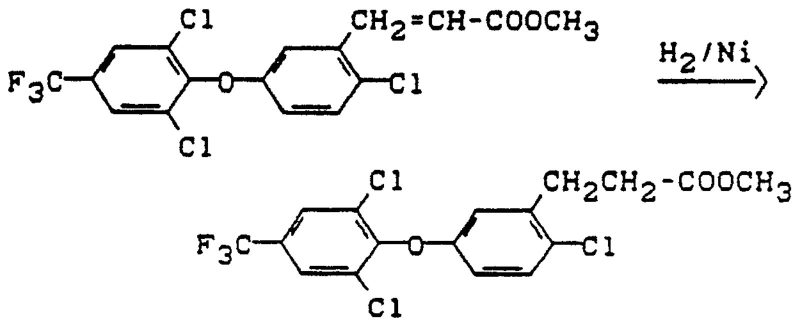


Verwendet man für das erfindungsgemäße Verfahren (d) beispielsweise 3-(2-Fluor-5-(2-chlor-4-trifluormethylphenoxy)-phenyl)-propionsäurepropylester und N-Bromsuccinimid als Ausgangsstoffe, so kann der Reaktionsablauf durch das folgende Formelschema skizziert werden:

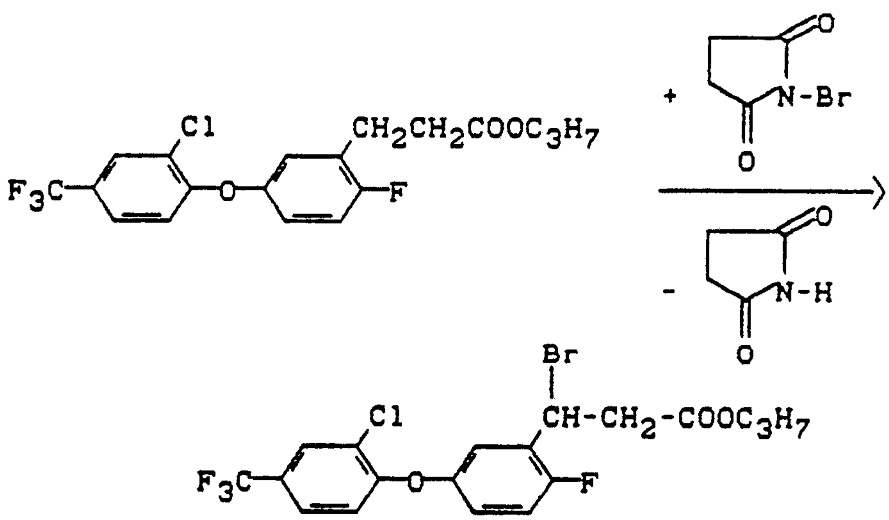

Verwendet man für das erfindungsgemäße Verfahren (e) beispielsweise 3-(2-Chlor-5-(2,6-dichlor-4-trifluormethyl-phenoxy)-phenyl)-propionsäurenitril und Natronlauge als Ausgangsstoffe, so kann der Reaktionsablauf durch das folgende Formelschema wiedergegeben werden:

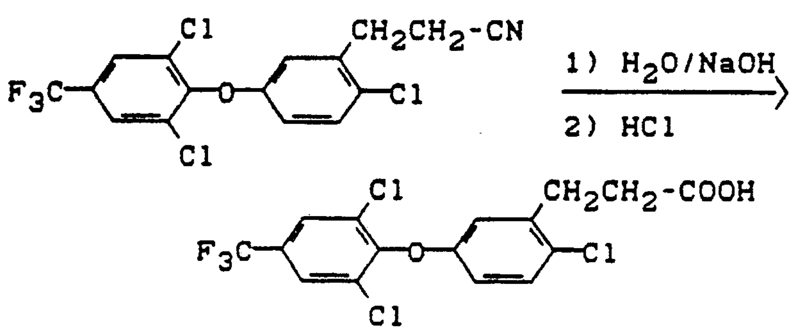


Verwendet man für das erfindungsgemäße Verfahren (f) beispielsweise 3-(2-Brom-5-(2-chlor-4-trifluormethylphenoxy)-phenyl)-propionsäure und Thionylchlorid als Ausgangsstoffe, so kann der Reaktionsablauf durch das folgende Formelschema wiedergegeben werden:

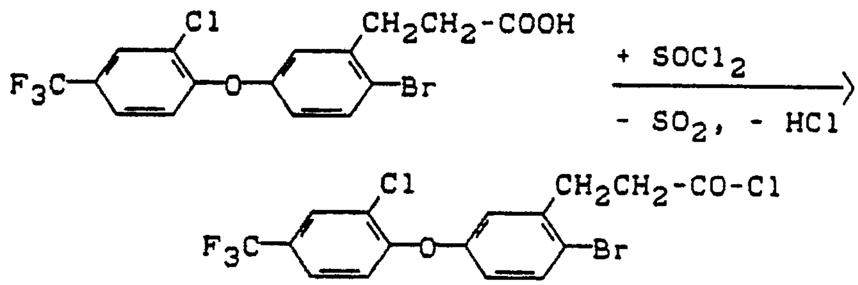


Verwendet man für das erfindungsgemäße Verfahren (g) beispielsweise 3-(2-Chlor-5-(2,6-dichlor-4-trifluormethyl-phenoxy)-phenyl)-propionsäurechlorid und Mercaptoessigsäuremethylester als Ausgangsstoffe, so kann der Reaktionsablauf durch das folgende Formelschema wiedergegeben werden:

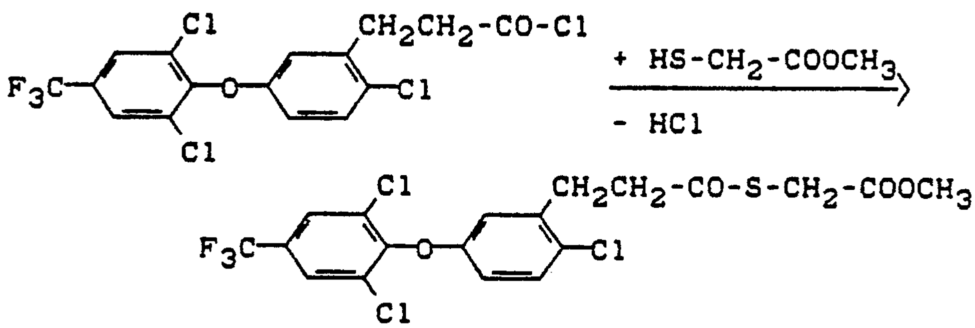


Verwendet man für das erfindungsgemäße Verfahren (h) beispielsweise 3-(3-(2,3,6-Trichlor-4-trifluormethylphenoxy)-phenyl)-propionsäure-isopropylester und Chlor als Ausgangsstoffe, so kann der Reaktionsablauf durch das folgende Formelschema wiedergegeben werden:

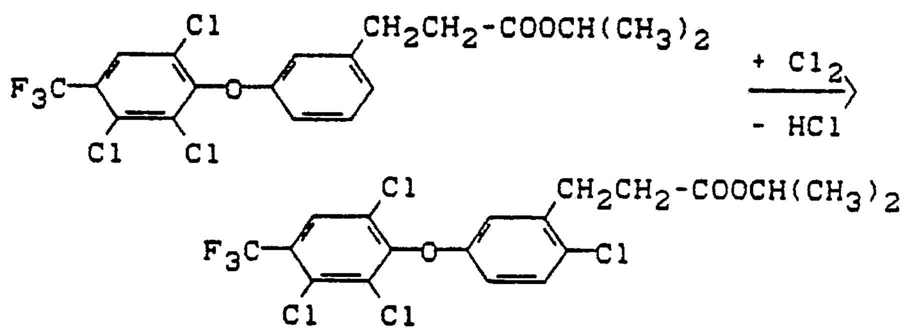


Die beim erfindungsgemäßen Verfahren (a) zur Herstellung von Verbindungen der Formel (I) als Ausgangstoffe zu verwendenden Phenoxyphenylaminoverbindungen sind durch die Formel (II) allgemein definiert. In Formel (II) haben $R^1$, $R^2$, $R^3$, $R^4$, $R^5$, $R^6$ und X vorzugsweise bzw. insbesondere diejenigen Bedeutungen. die bereits im Zusammenhang mit der Beschreibung der erfindungsgemäßen Ver bindungen der Formel (I) vorzugsweise bzw. als insbesondere bevorzugt für $R^1$, $R^2$, $R^3$, $R^4$, $R^5$, $R^6$ und X angegeben wurden.

Als Beispiele für die Ausgangsstoffe der Formel (II) seien genannt:
2-Chlor-5-(2-Chlor-4-trifluormethyl-phenoxy)-anilin, 2-Chlor-5-(2,6-dichlor-4-trifluormethyl-phenoxy-anilin, 2-Chlor-5-(2-chlor-6-fluor-4-trifluormethyl-phenoxy)-anilin, 2,4-Dichlor-5-(2-chlor-4-trifluormethyl-phenoxy)-anilin, 2,4-Dichlor-5-(2,6-dichlor-4-trifluormethyl-phenoxy)-anilin, 2,4-Dichlor-5-(2-chlor-6-fluor-4-trifluormethyl-phenoxy)-anilin, 2-Chlor-5-(2,3,6-trichlor-4-trifluormethyl-phenoxy)-anilin und 2-Chlor-5-(2,6-dichlor-3-fluor-4-trifluormethyl-phenoxy)-anilin.

Die Ausgangsstoffe der Formel (II) sind noch nicht aus der Literatur bekannt. Man erhält die Verbindungen der Formel (II), wenn man entsprechende Halogen-benzol-Derivate der allgemeinen Formel (IX)

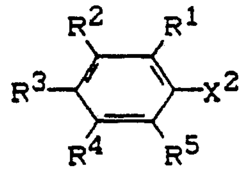

(IX)

in welcher
$R^1$, $R^2$, $R^3$, $R^4$ und $R^5$ die oben angegebenen Bedeutungen haben und
$X^2$ für Fluor oder Chlor steht,
mit Aminophenolen der allgemeinen Formel (X)

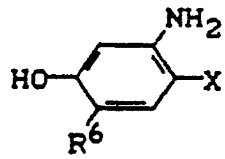

(X)

in welcher
$R^6$ und X die oben angegebenen Bedeutungen haben,
in Gegenwart eines Säureakzeptors, wie z. B. Natrium-oder Kalium-hydroxid, und in Gegenwart eines Verdünnungsmittels, wie z. B. Dimethylsulfoxid, bei Temperaturen zwischen 20 °C und 150 °C umsetzt und nach üblichen Methoden aufarbeitet.

Die als Zwischenprodukte zu verwendenden Halogenbenzol-Derivate sind durch die Formel (IX) allgemein definiert. In Formel (IX) haben $R^1$, $R^2$, $R^3$, $R^4$ und $R^5$ vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden und $X^2$ steht vorzugsweise für Chlor oder Fluor.

Als Beispiele für die Zwischenprodukte der Formel (IX) seien genannt:
3,4-Dichlor-benzotrifluorid, 3,4,5-Trichlor-benzotrifluorid, 3,4-Dichlor-5-fluor-benzotrifluorid, 2,3,4,5-Tetrachlor-benzotrifluorid, 3,5-Dichlor-2,4-difluor-benzotrifluorid und 3-Chlor-4,5-difluor-benzotrifluorid.

Die Verbindungen der Formel (IX) sind bekannt und/oder können nach an sich bekannten Verfahren hergestellt werden (vgl. J. Chem. Soc. 1969, 211-217; ibid. 1971, FR-A 2 538 380 (Chem. Abstracts 102 (1985), 61914x); EP-A 180 057; US-P 4 388 472 und vgl. Herstellungsbeispiele).

Die weiter als Zwischenprodukte benötigten Aminophenole sind durch die Formel (X) allgemein definiert. In Formel (X) haben $R^6$ und X vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt angegeben wurden.

Als Beispiele für die Zwischenprodukte der Formel (X) seien genannt:
3-Amino-4-chlor-phenol, 3-Amino-4,6-dichlor-phenol, 3-Amino-4-bromphenol und 3-Amino-4-fluor-phenol.

Die Verbindungen der Formel (X) sind bekannte organische Synthesechemikalien.

Die beim erfindungsgemäßen Verfahren (a) weiter als Ausgangsstoffe zu verwendenden Acrylsäure-Derivate sind durch die Formel (IV) allgemein definiert. In Formel (IV) haben $R^8$ und $R^{10}$ vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^8$ und $R^{10}$ angegeben wurden.

Als Beispiele für die Ausgangsstoffe der Formel (IV) seien genannt:
Acrylsäure-methylester und -ethylester, 2-Chlor-acrylsäure-methylester und -ethylester sowie 2-Brom-acrylsäure-methylester und -ethylester.

Die Verbindungen der Formel (IV) sind bekannte organische Synthesechemikalien.

Verfahren (a) wird unter Einsatz eines Hydrogenhalogenids ($HX^1$) durchgeführt. Als Beispiele hierfür seien Hydrogenfluorid, Hydrogenchlorid, Hydrogenbromid und Hydrogeniodid genannt. Hydrogenchlorid wird vorzugsweise eingesetzt.

Verfahren (a) wird vorzugsweise unter Verwendung eines organischen Lösungsmittels durchgeführt. Insbesondere geeignet sind Ether, wie z. B. Glycoldimethylether und Diglycoldimethylether, Tetrahydrofuran und Dioxan, Ketone, wie z. B. Aceton und Methylethylketon, sowie Amide wie z. B. Dimethylformamid.

Verfahren (a) wird weiter vorzugsweise in Gegenwart von Katalysatoren duchgeführt. Als solche

kommen insbesondere Kupfer und Kupferverbindungen, wie z. B. Kupfer(I)-chlorid, Kupfer(II)-chlorid, Kupfer(I)-bromid, Kupfer (I)-iodid, Kupfer(II)-sulfat und Kupfer(II)-nitrat in Betracht.

Die Reaktionstemperaturen können bei dem erfindungsgemäßen Verfahren (a) in einem größeren Bereich variiert werden. Im allgemeinen arbeitet man bei Temperaturen zwischen -20 °C und +80 °C, vorzugsweise bei Temperaturen zwischen 0 °C und 60 °C.

Verfahren (a) wird im allgemeinen bei Normaldruck durchgeführt.

Zur Durchführung des erfindungsgemäßen Verfahrens (a) setzt man je Mol Phenoxyphenylaminoverbindung der Formel (II) im allgemeinen zwischen 0,8 und 2,5 Mol, vorzugsweise zwischen 1,1 und 2,0 Mol, Natriumnitrit oder Kaliumnitrit, zwischen 2 und 50 Mol, vorzugsweise zwischen 5 und 25 Mol, Hydrogenhalogenid, und zwischen 1 und 3 Mol, vorzugsweise zwischen 1,5 und 2,5 Mol, Acrylsäure-Derivat der Formel (IV) ein.

Verfahren (a) kann unter den üblichen Bedingungen der "Meerwein-Arylierung" durchgeführt werden. In einer bevorzugten Ausführungsform von Verfahren (a) wird zunächst die Ausgangsverbindung der Formel (II) in einem Verdünnungsmittel, welches zumindest Wasser und ein Hydrogenhalogenid enthält, verrührt und unter Kühlen mit einer wäßrigen Lösung von Natriumnitrit oder Kaliumnitrit diazotiert. Dann wird das Acrylsäure-Derivat der Formel (IV) und gegebenenfalls der Katalysator zum Reaktionsgemisch gegeben. Wenn - gegebenenfalls nach leich tem Erwärmen - die Stickstoff-Entwicklung abgeklungen ist, kann nach üblichen Methoden aufgearbeitet werden.

Beispielsweise wird das Reaktionsgemisch mit einem mit Wasser praktisch nicht mischbaren organischen Lösungsmittel, wie z. B. Diethylether, verdünnt, nach Durchschütteln die organische Phase abgetrennt, mit wäßriger Natriumhydrogencarbonat-Lösung und mit Wasser gewaschen, getrocknet und filtriert. Das nach Einengen des Filtrats als Rückstand erhaltene Rohprodukt der Formel (I) kann auf übliche Weise, beispielsweise durch Säulenchromatographie, gereinigt werden.

Die beim erfindungsgemäßen Verfahren (b) als Ausgangsstoffe zu verwendenden Verbindungen sind durch die Formel (I) mit der Maßgabe, daß $R^7$, $R^8$ und/oder $R^9$ für Halogen stehen, allgemein definiert. In diesem Fall haben die Reste $R^1$, $R^2$, $R^3$, $R^4$, $R^5$, $R^6$, $R^{10}$ und X vorzugsweise bzw. insbesondere diejenigen Bedeutungen, die bereits im Rahmen der Beschreibung der erfindungsgemäßen Verbindungen der Formel (I) vorzugsweise bzw. als insbesondere bevorzugt angegeben wurden und $R^7$, $R^8$ und/oder $R^9$ stehen vorzugsweise für Chlor oder Brom.

Als Beispiele für die Ausgangsstoffe zu Verfahren (b) seien genannt:
2-Chlor- und 2-Brom-3-(2-chlor-5-(2-chlor-4-trifluormethyl-phenoxy)-phenyl)-propionsäure-methylester und -ethylester, 2-Chlor- und 2-Brom-3-(2-chlor-5-(2,6-dichlor-4-trifluormethyl-phenoxy)-phenyl)-propionsäuremethylester und -ethylester, 2-Chlor- und 2-Brom-3-(2- chlor-5-(2-chlor-6-fluor-4-trifluormethyl-phenoxy)-phenyl)-propionsäure-methylester und -ethylester, 2-Chlor-und 2-Brom-3-(2-Chlor-5-(2,3,6-trichlor-4-trifluormethyl-phenoxy)-phenyl)-propionsäure-methylester und -ethylester sowie 2-Chlor- und 2-Brom-3-(2-Chlor-5-(2,6-dichlor-3-fluor-4-trifluormethyl-phenoxy)-phenyl)-propionsäure-methylester und -ethylester.

Die oben beschriebenen Ausgangsstoffe der Formel (I) für Verfahren (b) sind erfindungsgemäße, neue Verbindungen; sie können nach den erfindungsgemäßen Verfahren (a), (d) oder (h) hergestellt werden.

Verfahren (b) wird unter Verwendung eines Hydrierungskatalysators durchgeführt. Als Beispiele hierfür seien Raney-Nickel, Platin und Palladium genannt. Vorzugsweise wird Raney-Nickel für Verfahren (b) eingesetzt.

Verfahren (b) wird in Gegenwart eines Verdünnungsmittels durchgeführt. Vorzugsweise werden Alkohole, wie Methanol, Ethanol, Propanol oder Isopropanol, Ether, wie Glycoldimethylether, Diglycoldimethylether, Tetrahydrofuran oder Dioxan, oder Ester, wie Essigsäuremethylester oder Essigsäureethylester, als Lösungsmittel für Verfahren (b) eingesetzt.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Verfahren (b) wird im allgemeinen bei Normaldruck oder erhöhtem Druck bis etwa 200 bar, vorzugsweise bis etwa 100 bar, durchgeführt.

Verfahren (b) kann unter den bei katalytischen Hydrierungen üblichen Bedingungen durchgeführt werden. In einer bevorzugten Ausführungsform von Verfahren (b) wird die Ausgangsverbindung der Formel (I) mit dem Verdünnungsmittel und dem Katalysator vermischt und dann so lange Wasserstoff zudosiert, bis kein Verbrauch von Wasserstoff mehr festzustellen ist. Nach dem Ende der Hydrierung filtriert man das Reaktionsgemisch und erhält nach Einengen des Filtrats das Rohprodukt als Rückstand, welches auf übliche Weise, beispielsweise durch Säulenchromatographie, gereinigt werden kann.

Die beim erfindungsgemäßen Verfahren (c) als Ausgangsstoffe zu verwendenden Verbindungen sind durch die Formel (V) allgemein definiert. In Formel (V) haben die Reste $R^1$, $R^2$, $R^3$, $R^4$, $R^5$, $R^6$, $R^{10}$ und X

vorzugsweise bzw. insbesondere diejenigen Bedeutungen, die bereits im Rahmen der Beschreibung der erfindungsgemäßen Verbindungen der Formel (I) vorzugsweise bzw. als insbesondere bevorzugt für $R^1$, $R^2$, $R^3$, $R^4$, $R^5$, $R^6$, $R^{10}$ und X angegeben wurden.

Als Beispiele für die Ausgangsstoffe der Formel (V) seien genannt:

3-(2-Chlor-5-(2-chlor-4-trifluormethyl-phenoxy)-phenyl)-acrylsäure-methylester und -ethylester, 3-(2-Chlor-5-(2,6-dichlor-4-trifluormethyl-phenoxy)-phenyl)-acrylsäu re-methylester und- ethylester, 3-(2-Chlor-5-(2-chlor-6-fluor-4-trifluormethyl-phenoxy)-phenyl)-acrylsäure-methylester und -ethylester, 3-(2-Chlor-5-(2,3,6-trichlor-4-trifluormethyl-phenoxy)-phenyl)-acrylsäure-methylester und -ethylester sowie 3-(2-Chlor-5-(2,6-dichlor-3-fluor-4-trifluormethyl-phenoxy)-phenyl-acrylsäure-methylester und -ethylester.

Die Ausgangsstoffe der Formel (V) sind noch nicht aus der Literatur bekannt. Man erhält die Verbindungen der Formel (V), wenn man entsprechende Phenoxybenzaldehyde der allgemeinen Formel (XI)

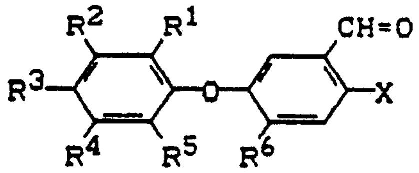

(XI)

in welcher

$R^1$, $R^2$, $R^3$, $R^4$, $R^5$, $R^6$ und X die oben angegebenen Bedeutungen haben,

mit einem geeigneten Carbonyl-Olefinierungsmittel, wie z. B. Malonsäure in Gegenwart von Pyridin oder Piperidin, bei Temperaturen zwischen 20 °C und 100 °C umsetzt, dann mit Wasser verdünnt und das kristallin anfallende Produkt der allgemeinen Formel (Va)

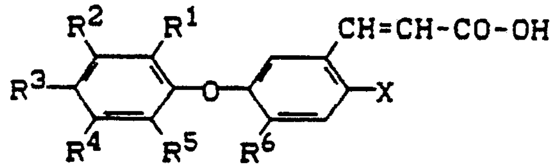

(Va)

in welcher

$R^1$, $R^2$, $R^3$, $R^4$, $R^5$, $R^6$ und X die oben angegebenen Bedeutungen haben,

durch Absaugen isoliert und gegebenenfalls nach üblichen Methoden in entsprechende Säurehalogenide, Ester und weitere Derivate gemäß Formel (V) umwandelt (vgl. DE-OS 30 44 810).

Die als Zwischenprodukte zu verwendenden Phenoxybenzaldehyde sind durch die Formel (XI) allgemein definiert. In Formel (XI) haben $R^1$, $R^2$, $R^3$, $R^4$, $R^5$, $R^6$ und X vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$, $R^6$ und X angegeben wurden.

Als Beispiele für die Zwischenprodukte der Formel (XI) seien genannt:

2-Chlor-5-(2-chlor-4-trifluormethyl-phenoxy)-benzaldehyd, 2-Chlor-5-(2,6-dichlor-4-trifluormethyl-phenoxy-benzaldehyd, 2-Chlor-5-(2-chlor-6-fluor-4-trifluormethyl-phenoxy)-benzaldehyd, 2-Chlor-5-(2,3,6-trichlor-4-trifluormethyl-phenoxy)-benzaldehyd und 2-Chlor-5-(2,6-dichlor-3-fluor-4-trifluormethyl-phenoxy)-benzaldehyd.

Die Phenoxybenzaldehyde der Formel (XI) sind bekannt und/oder können nach an sich bekannten Verfahren hergestellt werden (vgl. DE-OS 30 17 795/US 4 344 789).

Beim erfindungsgemäßen Verfahren (c) gelten bezüglich des zu verwendenden Hydrierungskatalysators und des Verdünnungsmittels sowie hinsichtlich Reaktionstemperatur, Druck, Verfahrensdurchführung und Aufarbeitung die entsprechenden obigen Angaben für Verfahren (b).

Die beim erfindungsgemäßen Verfahren (d) als Ausangsstoffe zu verwendenden Verbindungen sind durch die Formel (I) mit der Maßgabe, daß $R^7$ für Wasserstoff steht, allgemein definiert. In diesem Fall haben die Reste $R^1$, $R^2$, $R^3$, $R^4$, $R^5$, $R^6$, $R^8$, $R^9$ und $R^{10}$ vorzugsweise bzw. insbesondere diejenigen Bedeutungen, die bereits oben im Rahmen der Beschreibung der erfindungsgemäßen Verbindungen Formel (I) vorzugsweise bzw. als insbesondere bevorzugt für $R^1$, $R^2$, $R^3$, $R^4$, $R^5$, $R^6$, $R^8$, $R^9$ und $R^{10}$ angegeben wurden.

Als Beispiele für die Ausgangsstoffe zu Verfahren (d) seien genannt:

3-(2-Chlor-5-(2-chlor-4-trifluormethyl-phenoxy)-phenyl)-propionsäure-methylester und -ethylester, 3-(2-Chlor-5-(2,6-dichlor-4-trifluormethyl-phenoxy)-phenyl)-propionsäure-methylester und -ethylester, 3-(2-Chlor-5-(2-chlor-6-fluor-4-trifluormethyl-phenoxy)-phenyl)-propionsäure-methylester und -ethylester, 3-(2-Chlor-5-(2,3,6-trichlor-4-trifluormethyl-phenoxy)-phenyl)-propionsäure-methylesster und -ethylester sowie 3-(2-Chlor-5-(2,6-dichlor-3-fluor-4-trifluormethyl-phenoxy)-phenyl)-propionsäure-methylester und -ethylester.

Die vorausgehend beschriebenen Ausgangsstoffe der Formel (I) für Verfahren (d) sind erfindungsgemäße, neue Ver bindungen; sie können nach den erfindungsgemäßen Verfahren (b) und (c) hergestellt werden.

Die Reaktionstemperaturen können bei dem erfindungsgemäßen Verfahren (d) in einem größeren Bereich variiert werden. Im allgemeinen arbeitet man bei Temperaturen zwischen 0 °C und 150 °C, vorzugsweise bei Temperaturen zwischen 20 °C und 100 °C.

Verfahren (d) wird im allgemeinen bei Normaldruck durchgeführt.

Zur Durchführung des erfindungsgemäßen Verfahrens (d) werden die Ausgangsverbindung der Formel (I) und das N-Brom- bzw. N-Chlor-succinimid im allgemeinen in angenähert äquimolaren Mengen eingesetzt. Die Reaktionskomponenten werden im allgemeinen bei Raumtemperatur zusammen gegeben und das Reaktionsgemisch wird - gegebenenfalls bei erhöhter Temperatur - bis zum Ende der Umsetzung gerührt.

Nach Filtration und Einengen des Filtrats erhält man das Produkt der Formel (I) als Rückstand, welcher auf übliche Weise gereinigt werden kann.

Die beim erfindungsgemäßen Verfahren (e) als Ausgangsstoffe zu verwendenden Verbindungen sind durch die Formel (VI) allgemein definiert. In Formel (VI) haben die Reste $R^1$, $R^2$, $R^3$, $R^4$, $R^5$, $R^6$, $R^7$, $R^8$, $R^9$ und X vorzugsweise bzw. insbesondere diejenigen Bedeutungen, die bereits oben im Rahmen der Beschreibung der erfindungsge mäßen Verbindungen der Formel (I) vorzugsweise bzw. als insbesondere bevorzugt angegeben wurden und Y steht für Cyano, Methoxycarbonyl oder Ethoxycarbonyl.

Als Beispiele für die Ausgangsstoffe der Formel (VI) seien genannt:

2-Chlor- und 2-Brom-3-(2-chlor-5-(2-chlor-4-trifluormethyl-phenoxy)-phenyl)-propionitril, 2-Chlor- und 2-Brom-3-(2-chlor-5-(2,6-dichlor-4-trifluormethyl-phenoxy)-phenyl)-propionitril, 2-Chlor- und 2-Brom-3-(2-chlor-5-(2-chlor-6-fluor-4-trifluormethyl-phenoxy)-phenyl)-propionitril, 2-Chlor- und 2-Brom-3-(2-chlor-5-(2,3,6-trichlor-4-trifluormethyl-phenoxy)-phenyl)-propionitril, 2-Chlor- und 2-Brom-3-(2-chlor-5-(2,6-dichlor-3-fluor-4-trifluormethyl-phenoxy)-phenyl)-propionitril, 3-(2-Chlor-5-(2-chlor-4-trifluormethyl-phenoxy)-phenyl)-propionitril, 3-(2-Chlor-5-(2-chlor-6-fluor-4-trifluormethyl-phenoxy)-phenyl)-propionitril, 3-(2-Chlor-5-(2,6-dichlor-4-trifluormethyl-phenoxy)-phenyl)-propionitril, 3-(2-Chlor-5-(2,3,6-trichlor-4-trifluormethyl-phenoxy)-phenyl)-propionitril sowie 3-(2-Chlor-5-(2,6-dichlor-3-fluor-4-trifluormethyl-phenoxy)-phenyl)-propionitril.

Auch die bereits oben als Ausgangsstoffe für die Verfahren (b) und (d) einzeln aufgeführten Verbindungen der Formel (I) sind als Ausgangsstoffe für Verfahren (e) geeignet.

Die Ausgangsstoffe der Formel (VI) sind noch nicht aus der Literatur bekannt; sie können gemäß bzw. analog den erfindungsgemäßen Verfahren (a), (b), (c) und (d) hergestellt werden.

Verfahren (e) wird vorzugsweise in Gegenwart eines Verseifungshilfsmittels durchgeführt. Als solche kommen insbesondere starke Säuren, wie z. B. Salzsäure oder Schwefelsäure, oder Alkalihydroxide, wie z. B. Natriumhydroxid oder Kaliumhydroxid, in Betracht.

Verfahren (e) wird in Gegenwart von Wasser und gegebenenfalls in Gegenwart eines organischen Lösungsmittels durchgeführt. Als organische Lösungsmittel werden vorzugsweise Alkohole, wie z. B. Methanol oder Ethanol, eingesetzt.

Die Reaktionstemperaturen können bei dem erfindungsgemäßen Verfahren (e) in einem größeren Bereich variiert werden. Im allgemeinen arbeitet man bei Temperaturen zwischen 10 °C und 150 °C, vorzugsweise bei Temperaturen zwischen 20 °C und 100 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung von Verfahren (e) werden je Mol Ausgangsverbindung der Formel (VI) im allgemeinen zwischen 0.1 und 10 Mol, vorzugsweise zwischen 0,5 und 2 Mol, Verseifungshilfsmittel eingesetzt. Die Reaktionskomponenten werden im allgemeinen bei Raumtemperatur zusammen gegeben und das Reaktionsgemisch wird, gegebenenfalls bei erhöhter Temperatur, bis zum Reaktionsende gerührt. Das gegebenenfalls nach Einengen, Abkühlen und Ansäuern kristallin anfallende Reaktionsprodukt kann durch Absaugen isoliert werden.

Die beim erfindungsgemäßen Verfahren (f) als Ausgangsstoffe zu verwendenden Verbindungen sind durch die Formel (I) mit der Maßgabe, daß $R^{10}$ für Hydroxy steht, allgemein definiert. In diesem Fall haben die Reste $R^1$, $R^2$, $R^3$, $R^4$, $R^5$, $R^6$, $R^7$, $R^8$, $R^9$ und X vorzugsweise bzw. insbesondere diejenigen Bedeutungen, die bereits in Rahmen der Beschreibung der erfindungsgemäßen Verbindungen der Formel (I) vorzugsweise bzw. als insbesondere bevorzugt für $R^1$, $R^2$, $R^3$, $R^4$, $R^5$, $R^6$, $R^7$, $R^8$, $R^9$ und X angegeben wurden.

Als Beispiele für die Ausgangsstoffe zu Verfahren (f) seien genannt:
3-(2-Chlor-5-(2-chlor-4-trifluormethyl-phenoxy)-phenyl)-propionsäure, 3-(2-Chlor-5-(2,6-dichlor-4-trifluormethyl-phenoxy)-phenyl)-propionsäure, 3-(2-Chlor-5-(2-chlor-6-fluor-4-trifluormethyl-phenoxy)-phenyl)-propionsäure, 3-(2-Chlor-5-(2,3,6-trichlor-4-trifluormethylphenoxy)-phenyl)-propionsäure, 3-(2-Chlor-5-(2,6-dichlor-3-fluor-4-trifluormethyl-phenoxy)-phenyl)-propionsäure, 2-Chlor-und 2-Brom-3-(2-chlor-5-(2-chlor-4-trifluormethyl-phenoxy)-phenyl)-propionsäure, 2-Chlor- und 2-Brom-3-(2-chlor-5-(2,6-dichlor-4-trifluormethyl-phenoxy)-phenyl)-propionsäure, 2-Chlor- und 2-Brom-3-(2-chlor-5-(2-chlor-6-fluor-4-trifluormethyl-phenoxy)-phenyl)-propionsäure, 2-Chlor- und 2-Brom-3-(2-chlor-5-(2,3,6-trichlor-4-trifluormethyl-phenoxy)-phenyl)-propionsäure sowie 2-Chlor-und 2-Brom-3-(2-chlor-5-(2,6-di chlor-3-fluor-4-(trifluormethyl-phenoxy)-phenyl)-propionsäure.

Die Ausgangsstoffe der Formel (I) für Verfahren (f) sind erfindungsgemäße, neue Verbindungen : sie können nach dem erfindungsgemäßen Verfahren (e) hergestellt werden.

Verfahren (f) wird unter Verwendung eines Halogenierungsmittels durchgeführt. Es können die üblichen Mittel für die Umsetzung von Carbonsäuren zu Carbonsäurehalogeniden eingesetzt werden. Als Beispiele hierfür seien Phosgen, Thionylchlorid, Phosphorylchlorid und Benzotrichlorid genannt. Vorzugsweise wird Thionylchlorid als Halogenierungsmittel verwendet.

Verfahren (f) wird gegebenenfalls in Gegenwart eines Katalysators durchgeführt. Es können die für die Herstellung von Säurechloriden aus Säuren üblichen Katalysatoren, wie z. B. Pyridin oder Dimethylformamid, verwendet werden.

Verfahren (f) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsgemäßen Verfahren (f) in einem größeren Bereich variiert werden. Im allgemeinen arbeitet man bei Temperaturen zwischen 0 °C und 100 °C, vorzugsweise bei Temperaturen zwischen 10 °C und 90 °C.

Verfahren (f) wird im allgemeinen bei Normaldruck durchgeführt.

Zur Durchführung von Verfahren (f) werden je Mol Ausgangsverbindung der Formel (I) im allgemeinen zwischen 1 und 100 Mol, vorzugsweise zwischen 2 und 50 Mol, Halogenierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann durch Umkristallisation gereinigt werden, aber auch ohne weitere Reinigung für Folgeumsetzungen eingesetzt werden.

Die beim erfindungsgemäßen Verfahren (g) als Ausgangsstoffe zu verwendenden Verbindungen sind durch die Formel (I) mit der Maßgabe, daß $R^{10}$ für Halogen steht, allgemein definiert. In diesem Fall haben die Reste $R^1$, $R^2$, $R^3$, $R^4$, $R^5$, $R^6$, $R^7$, $R^8$, $R^9$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für $R^1$, $R^2$, $R^3$, $R^4$, $R^5$, $R^6$, $R^7$, $R^8$, $R^9$ und X angegeben wurden und $R^{10}$ steht vorzugsweise für Fluor, Chlor oder Brom insbesondere für Chlor.

Als Beispiele für die Ausgangsstoffe zu Verfahren (g) seien genannt:
3-(2-Chlor-5-(2-chlor-4-trifluormethyl-phenoxy)-phenyl)-propionsäure-chlorid, 3-(2-Chlor-5-(2,6-dichlor-4-trifluormethyl-phenoxy)-phenyl)-propionsäure-chlorid, 3-(2-Chlor-5-(2-chlor-6-fluor-4-trifluormethyl-phenoxy)-phenyl)-propionsäure-chlorid, 3-(2-Chlor-5-(2,3,6-trichlor-4-trifluormethyl-phenoxy)-phenyl)-propionsäure-chlorid, 3-(2-Chlor-5-(2,6-dichlor-3-fluor-4-trifluormethyl-phenoxy)-phenyl)-propionsäure-chlorid, 2-Chlor- und 2-Brom-3-(2-chlor-5-(2-chlor-4-trifluormethyl-phenoxy)-phenyl)-propionsäure-chlorid, 2-Chlor- und 2-Brom-3-(2-chlor-5-(2,6-dichlor-4-trifluormethyl-phenoxy)-phenyl)-propionsäure-chlorid, 2-Chlor- und 2-Brom-3-(2-chlor-5-(2-chlor-6-fluor-4-trifluormethyl-phenoxy)-phenyl)-propionsäure-chlorid, 2-Chlor- und 2-Brom-3-(2-chlor-5-(2,3,6-trichlor-4-trifluormethyl-phenoxy)-phenyl)-propionsäure-chlorid sowie 2-Chlor- und 2-Brom-3-(2-chlor-5-(2,6-dichlor-3-fluor-4-(trifluormethyl-phenoxy)-phenyl)-propionsäure-chlorid.

Die Ausgangsstoffe der Formel (I) für Verfahren (g) sind erfindungsgemäße, neue Verbindungen: sie können nach dem erfindungsgemäßen Verfahren (f) hergestellt werden.

Bei den als weitere Ausgangsstoffe für Verfahren (g) einzusetzenden Verbindungen der Formel (VII) hat $R^{10}$ vorzugsweise bzw. insbesondere diejenige Bedeutung, die bereits oben im Rahmen der Beschreibung der erfindungsgemäßen Verbindungen der Formel (I) vorzugsweise bzw. als insbesondere bevorzugt für $R^{10}$ angegeben wurde, wobei Halogen ausgenommen ist.

Als Beispiele für diese Ausgangsstoffe zu Verfahren (g) seien genannt:
Methylamin, Ethylamin, Propylamin, Isopropylamin, Cyanamid, Dimethylamin, Diethylamin, Hydroxylamin, O-Methylhydroxylamin, Hydrazin, Methylsulfonylhydrazin, Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthio-ethanol, 2-Benzyloxy-ethanol, 2-Benzylthio-ethanol, Hydroxymethanphosphonsäure-diethylester und -dimethylester, 1-Hydroxy-ethanphosphonsäure-dimethylester und -diethylester, 1-Hydroxy-1-phenyl-methanphosphonsäure-dimethylester und -diethylester, 3-Hydroxyfuran, Furfurylalkohol, Perhydrofurfurylalkohol, Milchsäure-methylester und -ethylester und Glycolsäure-methylester und -ethylester.

Diese Verbindungen sind bekannte Synthesechemikalien.

Das erfindungsgemäße Verfahren (g) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäureme thylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (g) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (g) in einem größeren Bereich variiert werden. Im allgemeinen arbeitet man bei Temperaturen zwischen 0 °C und 100 °C, vorzugsweise bei Temperaturen zwischen 10 °C und 50 °C.

Das erfindungsgemäße Verfahren (g) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (g) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (g) jeweils nach üblichen Methoden. Beispielsweise wird das Reaktionsgemisch - gegebenenfalls nach Einengen - mit Wasser verdünnt und das gewünschte Reaktionsprodukt wird mit einem mit Wasser praktisch nicht mischbaren organischen Lösungsmittel, z. B. Methylenchlorid, Chloroform, Diethylether, Toluol oder Xylol, extrahiert. Die organische Extraktionslösung wird mit Wasser gewaschen, mit einem üblichen Trockenmittel, wie z. B. Natriumsulfat, getrocknet und filtriert. Nach dem Einengen des Filtrats erhält man die Verbindungen der Formel (I) als Rohprodukte, welche auf übliche Weise, z. B. chromatographisch und/oder durch Umkristallisation gereinigt werden können.

Die beim erfindungsgemäßen Verfahren (h) als Ausgangsstoffe zu verwendenden Verbindungen sind durch die Formel (VIII) allgemein definiert. In Formel (VIII) haben die Reste $R^1$, $R^2$, $R^3$, $R^4$, $R^5$, $R^6$, $R^7$, $R^8$, $R^9$ und $R^{10}$ vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für $R^1$, $R^2$, $R^3$, $R^4$, $R^5$, $R^6$, $R^7$, $R^8$, $R^9$ und $R^{10}$ angegeben wurden.

Als Beispiele für die Ausgangsstoffe der Formel (VIII) seien genannt:

3-Brom-3-(2-chlor-5-(2,6-dichlor-4-trifluormethyl-phenoxy)-propionsäure sowie deren Methylester und Ethylester, 3-(2-Chlor-5-(2-chlor-4-trifluormethyl-phenoxy)-phenyl)-propionsäure sowie deren Methylester und Ethylester, 3-(2-Chlor-5-(2,6-dichlor-4-trifluormethyl-phenoxy)-phenyl)-propionsäure sowie deren Methylester und Ethylester, 3-(2-Chlor-5-(2-chlor-6-fluor-4-trifluormethyl-phenoxy)-phenyl)-propionsäure sowie deren Methylester und Ethylester, 3-(2-Chlor-5-(2,3,6-trichlor-4-trifluormethyl-phenoxy)-phenyl)-propionsäure sowie deren Methylester und Ethylester sowie 3-(2-Chlor-5-(2,6-dichlor-3-fluor-4-trifluormethyl-phenoxy)-phenyl)-propionsäure sowie deren Methyl- und Ethylester.

Die Ausgangsstoffe der Formel (VIII) sind bekannt und/oder können nach an sich bekannten Verfahren hergestellt werden (vgl. DE-OS 34 19 952; DE-OS 34 19 937).

Das erfindungsgemäße Verfahren (h) wird vorzugsweise unter Verwendung von Verdünnungsmitteln durchgeführt. Es kommen vor allem diejenigen organischen Lösungsmittel in Betracht, die bereits oben für

Verfahren (g) angegeben wurden, daneben jedoch auch Essigsäure und/oder Wasser.

Die Reaktionstemperaturen können bei dem erfindungsgemäßen Verfahren (h) in einem größeren Bereich variiert werden. Im allgemeinen arbeitet man bei Temperaturen zwischen 0 °C und 150 °C, vorzugsweise bei Temperaturen zwischen 20 °C und 120 °C.

Verfahen (h) wird im allgemeinen bei Normaldruck durchgeführt.

Zur Durchführung des erfindungsgemäßen Verfahrens (h) setzt man je Mol Ausgangsverbindung der Formel (VIII) im allgemeinen zwischen 1 und 5 Mol, vorzugsweise zwischen 1 und 3 Mol Chlor oder Brom ein.

Vorzugsweise wird die Ausgangsverbindung der Formel (VIII) in einem Verdünnungsmittel vorgelegt und das Chlor oder das Brom wird langsam - gegebenenfalls bei erhöhter Temperatur - eindosiert. Das Reaktionsgemisch wird bis zum Ende der Umsetzung gerührt und anschließend eingeengt. Der Rückstand wird in einem mit Wasser praktisch nicht mischbaren organischen Lösungsmittel, wie z. B. Methylenchlorid, gelöst; diese Lösung wird mit Wasser gewaschen, mit einem üblichen Trockenmittel, wie z. B. Natriumsulfat, getrocknet und filtriert. Das Filtrat wird eingeengt, der Rückstand durch Verreiben mit einem geeigneten Lösungsmittel, wie z. B. Petrolether, zur Kristallisation gebracht und das kristalline Produkt wird durch Absaugen isoliert.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich insbesondere zur selektiven Bekämpfung von dikotylen Unkräutern in monokotylen Kulturen im Nachauflaufverfahren.

Die erfindungsgemäßen Wirkstoffe greifen in den Metabolismus der Pflanzen ein und können deshalb

als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesent lichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.,B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zut Unkrautbekämpfung in Zuckerrüben und 4-Amino-6-(1,1-dimethylethyl)-3-methylthio-1,2,4-triazin-5(4H)-on (METRIBUZIN) zur Unkraut bekämpfung in Sojabohnen, in Frage; ferner auch 2,4-Dichlorphenoxyessigsäure (2,4-D); 4-(2,4-Dichlorphenoxy)buttersäure (2,4-DB); 2,4-Dichlorphenoxypropionsäure (2,4-DP); 2-Chlor-4-ethylamino-6-isopropylamino-1,3,5-triazin (ATRAZIN); 3-Isopropyl-2,1,3-benzothiadiazin-4-on-2,2-dioxid (BENTAZON); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 3,6-Dichlor-2-pyridincarbonsäure (CLOPYRALID); 2-{4-[(6-Chlor-2-benzoxazolyl)-oxy]-phenoxy}-propansäure, deren Methyl- oder deren Ethylester (FENOXAPROP); 2-[4-(3,5-Dichlorpyrid-2-yloxy)-phenoxy]-propionsäure-(trimethylsilylmethylester); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yl-oxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON) und 3,5,6-Trichlor-2-pyridyloxyessigsäure (TRICLOPYR).

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

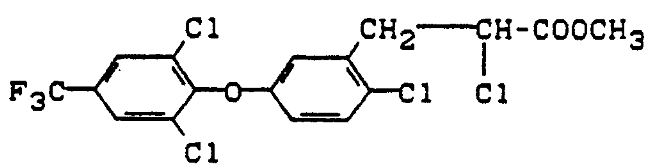

**(Verfahren (a))**

14,3 g (0,04 Mol) 2-Chlor-5-(2,6-dichlor-4-trifluormethyl-phenoxy)-anilin werden in einer Mischung aus 150 ml Aceton und 90 ml konzentrierter Salzsäure gelöst, die Lösung auf 0 °C abgekühlt und durch Zugabe von 4,8 g (0,07 Mol) Natriumnitrit in 10 ml Wasser diazotiert. Man läßt noch 10 Minuten nachrühren und gibt dann nacheinander 6,9 g (0,08 Mol) Acrylsäuremethylester und eine Lösung von 2,0 g Kupfer(I)-chlorid und 0,2 g Kupfer(II)-chlorid in 10 ml halbkonzentrierter Salzsäure zu. Das Reaktionsgemisch wird auf 40 °C erwärmt, wobei Stickstoff entweicht. Wenn die Stickstoffentwicklung beendet ist, wird der Ansatz mit Ether verdünnt und mit 5 %iger Natriumhydrogencarbonatlösung und mit Wasser gewaschen. Die organische Phase wird abgetrennt, über Natriumsulfat getrocknet, filtriert und eingeengt. Der verbleibende Rückstand wird zur Reinigung mit Essigester/n-Hexan 1 : 4 als Laufmittel über eine Kieselgelsäule chromatografiert.

Man erhält 9,9 g (54 % der Theorie) 2-Chlor-3-[3-(2,6-dichlor-4-trifluormethyl-phenoxy)-6-chlor-phenyl]-propionsäure-methylester als gelbes Öl.
$^1$H-NMR ($CDCl_3$) 300 MHz, TMS als innerer Standard
δ-Werte: 3,22 (d,d $J_1$ = 15 Hz, $J_2$ = 7,5 Hz, 1H); 3,45 (d,d $J_1$ = 15 Hz, $J_2$ = 7,5 Hz, 1H); 3,75 (s, 3H); 4,5 (pseudo-t J = 7,5 Hz, 1H); 6,73 (m, 2H); 7,3 (d, J = 9 Hz, 1H); 7,68 (s, 2H).

Beispiel 2

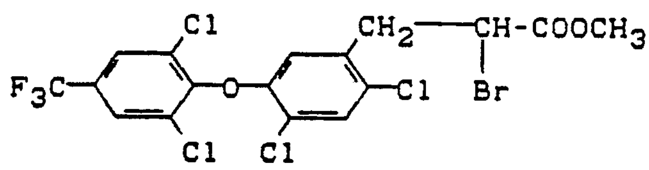

(Verfahren (a))

9,8 g (0,025 Mol) 2,4-Dichlor-5-(2,6-dichlor-4-trifluormethyl-phenoxy)-anilin werden in einer Mischung aus 100 ml Aceton und 60 ml 48 %iger wäßriger Bromwasserstoffsäure gelöst, die Lösung auf 0 °C abgekühlt und durch Zugabe von 2,4 g (0,035 Mol) Natriumnitrit, gelöst in 5 ml Wasser, diazotiert. Man läßt noch 10 Minuten nachrühren und gibt dann nacheinander 4,3 g (0,05 Mol) Acrylsäuremethylester und eine Lösung von 1 g Kupfer(I)-bromid und 0,1 g Kupfer(II)-bromid in 5 ml 24 %iger Bromwasserstoffsäure zu. Das Reaktionsgemisch wird auf 40 °C erwärmt, wobei Stickstoff entweicht. Wenn die Stickstoffentwicklung beendet ist, wird der Ansatz mit Methylenchlorid verdünnt und mit 5 %iger Natriumhydrogencarbonatlösung und mit Wasser gewaschen. Die organische Phase wird abgetrennt, über Natriumsulfat getrocknet, filtriert und eingeengt. Der verbleibende Rückstand wird mit Essigester n-Hexan 1 : 4 als Laufmittel über eine Kieselgelsäule chromatografiert.

Man erhält 6,1 g (42,5 % der Theorie) 2-Brom-3-[3-(2,6-dichlor-4-trifluormethyl-phenoxy)-4,6-dichlorphenyl]-propionsäuremethylester als gelbes Öl.
$^1$H-NMR ($CDCl_3$) 300 MHz, TMS als innerer Standard
δ-Werte: 3,22 (d,d $J_1$ = 15 Hz, $J_2$ = 7,5 Hz, 1H); 3,40 (d,d, $J_1$ = 15 Hz, $J_2$ = 7,5 Hz, 1H); 3,70 (s, 3H); 4,45 (pseudo-t J = 7,5 Hz); 6,36 (s, 1H); 7,53 (s, 1H); 7,72 (s, 2H).

Beispiel 3

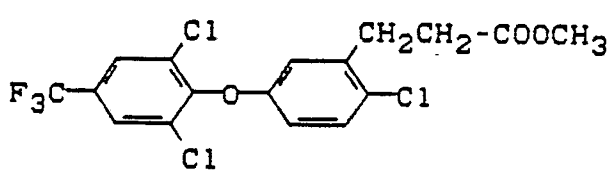

(Verfahren (b))

6,9 g (0,015 Mol) 2-Chlor-3-[3-(2,6-dichlor-4-trifluormethyl-phenoxy)-6-chlor-phenyl]-propionsäuremethylester werden in 200 ml Methanol gelöst und unter Zusatz von 5 g Raney-Nickel bei 40 °C unter 90 - 100 bar Wasserstoff-Druck 2 Stunden hydriert. Nach dem Abfiltrieren des Katalysators wird die Lösung eingeengt und der Rückstand durch Chromatographie über eine Kieselgelsäule mit Methylenchlorid als Laufmittel gereinigt.

Man erhält 4,4 g (69 % der Theorie) 3-[3-(2,6-dichlor-4- trifluormethyl-phenoxy)-6-chlor-phenyl]-propionsäuremethylester als farbloses Öl.

$^1H$-NMR ($CDCl_3$) 300 MHz, TMS als innerer Standard
$\delta$-Werte: 2,62 (t, J = 7,5 Hz, 2 H); 3,0 (t, J = 7,5 Hz, 2H); 3,67 (s, 3H); 6,63 (d,d, $J_1$ = 9 Hz, $J_2$ = 3,5 Hz, 1H); 6,72 (d, J = 3,5 Hz, 1H); 7,25 (d, J = 9 Hz, 1H); 7,68 (s, 2H).

Beispiel 4

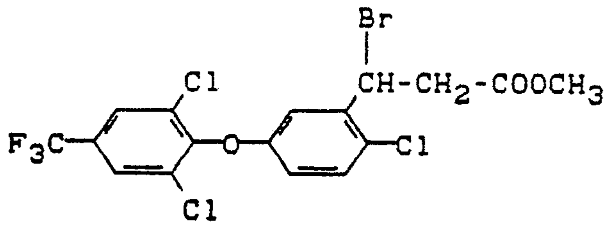

(Verfahren (d))

1,6 g (0,00375 Mol) 3-[3-(2,6-dichlor-4-trifluormethylphenoxy)-6-chlor-phenyl]-propionsäuremethylester werden in 50 ml Tetrachlorkohlenstoff gelöst, 0,67 g N-Bromsuccinimid und eine Spatelspitze 2,2'-Azoisobuttersäuredinitril zugegeben und das Reaktionsgemisch über Nacht unter Rückfluß gekocht.

Nach dem Abfiltrieren engt man die Tetrachlorkohlenstoff-Lösung ein und erhält 1,9 g (100 % der Theorie) 3-Brom-3-[3-(2,6-dichlor-4-trifluormethyl-phenoxy)-6-chlorphenyl]-propionsäuremethylester als gelbliches Öl.
$^1H$-NMR ($CDCl_3$), 300 MHz, TMS als innerer Standard,
$\delta$-Werte: 3,2 (d,d, $J_1$ = 16,5 Hz, $J_2$ = 7,5 Hz, 1H); 3,3 (d,d, $J_1$ = 16,5 Hz, $J_2$ = 9 Hz, 1H); 3,72 (s, 3H); 5,78 (d,d, $J_1$ = 9,0 Hz, $J_2$ = 7,5 Hz, 1H); 6,65 (d,d, $J_1$ = 10 Hz, $J_2$ = 3,5 Hz, 1H); 7,1 (d, J = 3,5 Hz, 1H); 7,3 (d, J = 10 Hz, 1H; 7,7 (s, 2H).

Beispiel 5

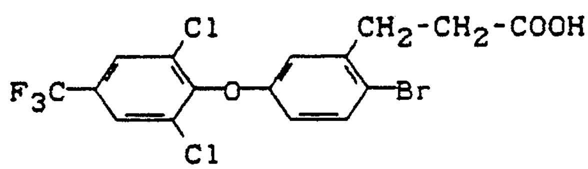

(Verfahren (h))

36,5 g (0,093 Mol) 3-[3-(2,6-Dichlor-4-trifluormethylphenoxy)-phenyl]-propionsäuremethylester werden in 200 ml Eisessig gelöst und bei Siedetemperatur 32 g (0,2 Mol) Brom zugetropft. Das Reaktionsgemisch wird 16 Stunden unter Rückfluß gekocht, unter vermindertem Druck eingeengt und der Rückstand in Methylenchlorid aufgenommen. Die Methylenchloridlösung wird mehrmals mit Wasser gewaschen, über Natriumsulfat getrocknet, filtriert und eingeengt. Der Rückstand wird mit Petrolether auskristallisiert, abgesaugt und an der Luft getrocknet. Das so erhaltene Rohprodukt wird zur Reinigung aus n-Hexan/Essigsäureethylester im Verhältnis 3 : 1 umkristallisiert.

Man erhält 3,2 g (75 % der Theorie) 3-[3-(2,6-Dichlor-4-trifluormethyl-phenoxy)-6-brom-phenyl]-propionsäure vom Schmelzpunkt 180 °C.

Analog zu den Beispielen 1, 2, 3, 4 und 5 sowie nach der allgemeinen Beschreibung der erfindungsgemäßen Herstellungsverfahren können die in der nachstehenden Tabelle 1 aufgeführten Verbindungen der Formel (I) erhalten werden.

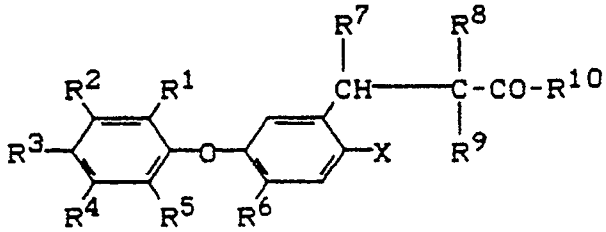

(I)

Die $^1$H-NMR-Spektren wurden aufgenommen in $CDCl_3$ mit Tetramethylsilan als inneren Standard. Angegeben sind unter "Physikal. Daten" in der Regel die chemischen Verschiebungen als $\delta$-Werte für die Wasserstoffatome aus der Gruppierung $CR^8R^9$.

**Tabelle 1**: Beispiele für Verbindungen der Formel (I)

| Beisp.-Nr. | $R^1$ | $R^2$ | $R^3$ | $R^4$ | $R^5$ | $R^6$ | $R^7$ | $R^8$ | $R^9$ | $R^{10}$ | X | Physikal. Daten |
|---|---|---|---|---|---|---|---|---|---|---|---|---|
| 6 | Cl | H | $CF_3$ | H | Cl | Cl | H | H | Cl | $OCH_3$ | Cl | δ = 4,58 (pseudo t) |
| 7 | Cl | H | $CF_3$ | H | H | H | H | Cl | Cl | $OCH_3$ | Cl | Öl |
| 8 | Cl | H | $CF_3$ | Cl | Cl | H | H | H | Cl | $OCH_3$ | Cl | δ = 4,48 (pseudo t) |
| 9 | Cl | H | $CF_3$ | F | Cl | H | H | H | Cl | $OCH_3$ | Cl | δ = 4,58 (pseudo t) |
| 10 | Cl | H | $CF_3$ | H | Cl | Cl | H | H | H | $OCH_3$ | Cl | δ = 2,54 (t) |
| 11 | Cl | H | $CF_3$ | F | Cl | H | H | H | H | $OCH_3$ | Cl | δ = 2,63 (t) |
| 12 | Cl | H | $CF_3$ | H | Cl | H | H | H | H | $OCH_3$ | Br | Fp. 53 °C |
| 13 | Cl | H | $CF_3$ | H | Cl | H | H | H | H | OH | Cl | Fp.152 °C |
| 14 | Cl | H | $CF_3$ | Cl | Cl | H | H | H | H | $OCH_3$ | Cl | |
| 15 | Cl | F | $CF_3$ | F | Cl | H | H | H | Cl | $OCH_3$ | Cl | δ = 4,58 (dd) |
| 16 | Cl | F | $CF_3$ | F | F | H | H | H | Cl | $OCH_3$ | Cl | |
| 17 | Cl | H | $CF_3$ | H | H | H | H | H | H | Cl | Cl | |
| 18 | Cl | H | $CF_3$ | H | H | H | H | H | H | OH | Cl | |
| 19 | Cl | H | $CF_3$ | H | Cl | H | H | H | H | Cl | Cl | |
| 20 | Cl | H | $CF_3$ | H | H | H | H | H | H | $OCH_3$ | Cl | |
| 21 | Cl | H | $CF_3$ | H | H | H | H | H | H | $OC_2H_5$ | Cl | Öl |
| 22 | Cl | Cl | $CF_3$ | H | Cl | H | H | H | H | $OC_3H_7$ | Cl | |

Tabelle 1 - Fortsetzung

| Beisp.-Nr. | $R^1$ | $R^2$ | $R^3$ | $R^4$ | $R^5$ | $R^6$ | $R^7$ | $R^8$ | $R^9$ | $R^{10}$ | X | Physikal. Daten |
|---|---|---|---|---|---|---|---|---|---|---|---|---|
| 23 | Cl | H | $CF_3$ | H | H | Cl | H | H | H | OH | Cl | |
| 24 | Cl | H | $CF_3$ | H | H | Cl | H | H | H | $OC_2H_5$ | Cl | |
| 25 | Cl | H | $CF_3$ | H | Cl | H | H | H | H | $OC_4H_9$ | Cl | |
| 26 | Cl | H | $CF_3$ | H | Cl | H | H | H | H | $OCH_2COOCH_3$ | Cl | |
| 27 | Cl | H | $CF_3$ | H | Cl | H | H | H | H | $OCHCOOCH_3$<br>\|<br>$CH_3$ | Cl | |
| 28 | Cl | H | $CF_3$ | H | H | Cl | H | H | H | $SCH_2COOCH_3$ | Cl | |
| 29 | Cl | Cl | $CF_3$ | H | Cl | H | H | H | Cl | $OCH_2CH_2OCH_3$ | Cl | |
| 30 | Cl | H | $CF_3$ | H | Cl | H | H | H | H | $OCH_2CH_2SCH_3$ | Cl | |
| 31 | Cl | H | $CF_3$ | H | Cl | H | H | H | H | O<br>‖<br>$OCH_2P(OC_2H_5)_2$ | Cl | |
| 32 | Cl | H | $CF_3$ | F | Cl | H | H | H | Cl | $SC_2H_5$ | Cl | |
| 33 | Cl | H | $CF_3$ | H | H | H | H | H | H | $NHCH(CH_3)_2$ | Cl | |
| 34 | Cl | H | $CF_3$ | H | Cl | H | H | H | H | $N(C_2H_5)_2$ | Cl | |
| 35 | Cl | H | $CF_3$ | H | Cl | H | H | H | H | $OCH_2CH_2SC_2H_5$ | Cl | |
| 36 | Cl | H | $CF_3$ | H | H | H | H | H | H | $NHSO_2CH_3$ | Cl | |

Tabelle 1 - Fortsetzung

| Beisp.-Nr. | $R^1$ | $R^2$ | $R^3$ | $R^4$ | $R^5$ | $R^6$ | $R^7$ | $R^8$ | $R^9$ | $R^{10}$ | X | Physikal. Daten |
|---|---|---|---|---|---|---|---|---|---|---|---|---|
| 37 | Cl | H | $CF_3$ | H | Cl | H | H | H | H | $OCH_2CH_2OC_2H_5$ | Cl | |
| 38 | Cl | H | $CF_3$ | H | H | H | H | H | H | $OCHCOOC_2H_5$<br>\|<br>$CH_3$ | Cl | |
| 39 | Cl | H | $CF_3$ | H | Cl | H | H | H | H | $OCH_2CH_2OCH_2$– 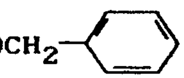 | Cl | |
| 40 | Cl | Cl | $CF_3$ | H | Cl | H | H | H | H | $OCH_2$ 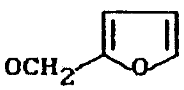 | Cl | |
| 41 | Cl | H | $CF_3$ | H | H | H | H | H | Cl | $NHOCH_3$ | Cl | |
| 42 | Cl | H | $CF_3$ | H | H | H | H | H | H | $OCH_2COOC_4H_9$ | Cl | |
| 43 | Cl | H | $CF_3$ | H | Cl | H | H | H | Cl | $NHCH_2COOC_2H_5$ | Cl | |
| 44 | Cl | Cl | $CF_3$ | H | Cl | H | H | H | H | $NHNHSO_2CH_3$ | Cl | |
| 45 | Cl | H | $F_3C-SO_2-$ | H | H | H | H | H | Cl | $OCH_3$ | Cl | $\delta$ = 4,62 (dd) |
| 46 | Cl | H | $F_3C-SO_2-$ | H | Cl | H | H | H | Cl | $OCH_3$ | Cl | $\delta$ = 4,58 (dd) |
| 47 | Cl | H | $CF_3$ | F | F | H | H | H | Cl | $OCH_3$ | Cl | $\delta$ = 4,58 (pseudo t) |
| 48 | Cl | H | $CF_3$ | H | F | H | H | H | Cl | $OCH_3$ | Cl | $\delta$ = 4,58 (pseudo t) |
| 49 | Cl | H | $CF_3$ | H | Cl | H | Br | H | H | $OCH_3$ | Br | $\delta$ = 3,20 (dd)und 3,26 (dd) |

Tabelle 1 - Fortsetzung

| Beisp.-Nr. | $R^1$ | $R^2$ | $R^3$ | $R^4$ | $R^5$ | $R^6$ | $R^7$ | $R^8$ | $R^9$ | $R^{10}$ | X | Physikal. Daten |
|---|---|---|---|---|---|---|---|---|---|---|---|---|
| 50 | F | H | $CF_3$ | H | F | H | H | H | Cl | $OCH_3$ | Cl | δ = 4,58 (pseudo t) |
| 51 | Cl | F | $CF_3$ | F | F | H | H | H | Cl | $OCH_3$ | Cl | δ = 4,58 (pseudo t) |
| 52 | Cl | F | $CF_3$ | F | Cl | H | H | H | H | $OCH_3$ | Cl | δ = 2,64 (t) |
| 53 | Cl | H | $CF_3$ | F | F | H | H | H | H | $OCH_3$ | Cl | δ = 2,63 (t) |
| 54 | Cl | H | $CF_3$ | H | F | H | H | H | H | $OCH_3$ | Cl | δ = 2,63 (t) |
| 55 | F | H | $CF_3$ | H | F | H | H | H | Br | $OCH_3$ | Cl | Öl |
| 56 | $CF_3$ | H | $CF_3$ | H | H | H | H | H | Cl | $OCH_3$ | Cl | Öl |
| 57 | Cl | H | $CF_3$ | H | Cl | H | H | H | H | $OC_2H_5$ | Cl | Fp. 50° C |
| 58 | Cl | H | $CF_3$ | H | H | H | H | H | H | $NHSO_2CH_3$ | Br | Fp. 153° C |
| 59 | Cl | H | $CF_3$ | Cl | Cl | H | H | H | H | $OC_2H_5$ | Cl | Öl |
| 60 | Cl | H | $CF_3$ | Cl | Cl | H | H | H | H | $OC_2H_5$ | Br | Fp. 64° C |
| 61 | Cl | H | $CF_3$ | H | H | H | H | H | H | $OC_2H_5$ | Br | Öl |
| 62 | Cl | H | $F_3C-SO_2-$ | H | F | H | H | H | Cl | $OCH_3$ | Cl | Öl |
| 63 | Cl | H | $CF_3$ | H | Cl | H | H | H | H | $SCH_2COOCH_3$ | Br | Fp. 66° C |
| 64 | Cl | H | $CF_3$ | H | Cl | H | H | H | H | $OCH_2COOC_4H_9$ | Br | Fp. 81° C |
| 65 | Cl | H | $CF_3$ | H | Cl | H | H | H | H | $OCH(CH_3)COOC_2H_5$ | Br | Öl |

Ausgangstoffe der Formel (II)

Beispiel (II-1)

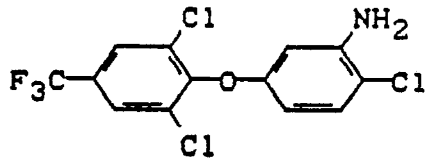

9,7 g (0,0676 Mol) 3-Amino-4-chlor-phenol, 16,7 g (0,0669 Mol) Trifluormethyl-3,4,5-trichlorbenzol und 2,7 g (0,0675 Mol) Natriumhydroxidplätzchen werden in 200 ml Dimethylsulfoxid 17 Stunden bei 100 °C gerührt. Nach dem abkühlen auf Raumtemperatur wird das Reaktionsgemisch in 250 ml Methylenchlorid aufgenommen und zweimal mit je 100 ml 1N-Natronlauge und anschließend mit Wasser gewaschen. Man trocknet die organische Phase über Natriumsulfat, filtriert und engt das Filtrat unter vermindertem Druck ein. Der Rückstand wird zur Reinigung über einen Kieselgelsäule mit Methylenchlorid als Laufmittel chromatographiert.

Man erhält 16,6 g (69,5 % der Theorie) 2-Chlor-5-(2,6-dichlor-4-trifluormethyl-phenoxy)-anilin als braunes Öl.

Analog Beispiel (II-1) können die in der nachstehenden Tabelle 2 aufgeführten Ausgangsstoffe der Formel (II) hergestellt werden.

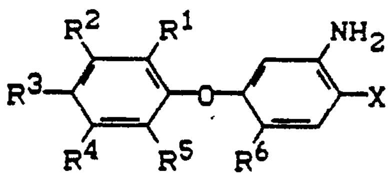

(II)

**Tabelle 2:** Beispiele für die Ausgangsstoffe der Formel (II)

| Beisp.-Nr. | $R^1$ | $R^2$ | $R^3$ | $R^4$ | $R^5$ | $R^6$ | X | Schmelzpunkt (°C) |
|---|---|---|---|---|---|---|---|---|
| II-2 | Cl | H | $CF_3$ | Cl | Cl | H | Cl | 87 |
| II-3 | Cl | H | $CF_3$ | F | Cl | H | Cl | 57 |
| II-4 | Cl | H | $CF_3$ | H | H | H | Cl | |
| II-5 | Cl | H | $CF_3$ | H | Cl | Cl | Cl | |
| II-6 | Cl | H | $CF_3$ | Cl | Cl | Cl | Cl | |
| II-7 | Cl | H | $CF_3$ | F | Cl | Cl | Cl | |
| II-8 | Cl | H | $CF_3$ | H | F | H | Cl | 60 |

Ausgangsstoffe der Formel VI

Beispiel (VI-1)

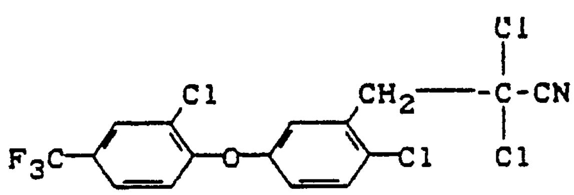

19,3 g (0,06 Mol) 2-Chlor-5-(2-chlor-4-trifluormethyl-phenoxy)-anilin werden in einer Mischung aus 150 ml Aceton, 60 ml konz. Salzsäure und 100 ml Wasser gelöst. Die Lösung wird auf 0° C abgekühlt und durch Zugabe von 6,2 g (0,09 Mol) Natriumnitrit in 10 ml Wasser diazotiert. Man gibt dann 10,5 g 2-Chloracrylsäurenitril (0,12 Mol), 2,0 g Kupfer(I)-chlorid und 0,2 g Kupfer-(II)chlorid in 10 ml halbkonzentrierter Salzsäure dazu. Das Reaktionsgemisch wird bis zum Ende der Stickstoffentwicklung auf 40° C erwärmt und wie in Beispiel 1 angegeben aufgearbeitet.

Man erhält 17,6 g (69 % der Theorie) 2,2-Dichlor-3-[3-(2-chlor-4-trifluormethyl-phenoxy)-6-chlor-phenyl]-propionsäurenitril als gelbes Öl

$^1$H-NMR ($CDCl_3$) 300 MHz, TMS als innerer Standard
δ-Werte: 7,78 (d, J = 3,5 Hz; 1H); 7,50 (m, 2H)
7,20 (d, J = 3,5 Hz; 1H); 7,05 (m, 2H); 3,98 (s, 2H).

Beispiel (IX-1) : (IX-2)

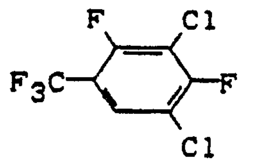

+

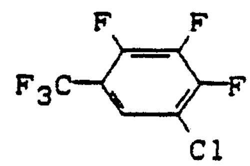

Zu 800 g (13,8 Mol) Kaliumfluorid in 1800 ml trockenem Tetramethylensulfon gibt man 900 g (3,17 Mol) 2,3,4,5-Tetrachlorbenzotrifluorid (vgl. z. B. EP 150 587) und erhitzt 10 Stunden auf 200 °C. Zur Aufarbeitung wird im Vakuum eingeengt und destilliert.

Man erhält zunächst 50 g (6,8 % der Theorie) an 2,3,4-Trifluor-5-chlor-benzotrifluorid vom Siedepunkt Kp. 31 -38 °C bei 10 mbar und vom Brechungsindex $n_D^{20}$ = 1,4130 und als 2. Fraktion 490 g (61,6 % der Theorie) an 2,4-Difluor-3,5-dichlorbenzotrifluorid vom Siedepunkt 57 -59 °C bei 10 mbar und vom Brechungsindex $n_D^{20}$ = 1,4510.

Beispiel (IX-3)

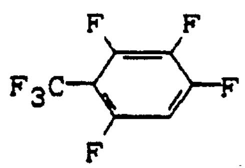

252,5 g (1 Mol) 3-Chlor-2,4,5,6-tetrafluorbenzotrifluorid (vergl. z. B. Zh. obshch. Khim. 37, 1686-1687 [1967]) und 86 g (1,05 Mol) Natriumacetat in 1000 ml Eisessig werden in Gegenwart von 10 g Palladium auf Aktivkohle (5 %ig) bei einem Wasserstoffdruck von 30 bis 50 bar und 120 °C bis zur Druckkonstanz hydriert. Zur Aufarbeitung filtriert man aus der erkalteten Reaktionsmischung den Katalysator ab und destilliert den Rückstand.

Man erhält 210 g (96 % der Theorie) an 2,3,4,6-Tetrafluorbenzotrifluorid vom Siedepunkt 105 - 106 °C und vom Brechungsindex $n_D^{20}$ = 1,3770.

In entsprechender Weise erhält man die folgenden Halogen-benzol-Derivate der Formel (IX):

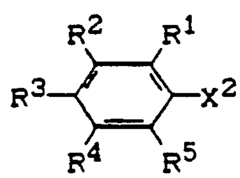

(IX)

| Beisp.-Nr. | 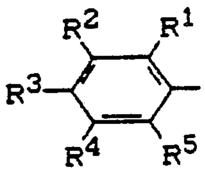 | $X^2$ | Siedepunkt / mbar Brechungsindex $n_D^{20}$ |
|---|---|---|---|
| IX-4 | 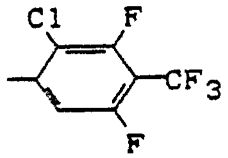 | F | Kp.: 145 °C/ 760<br>$n_D^{20}$ = 1,4170 |
| IX-5 | 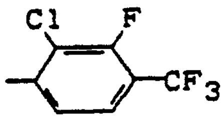 | F | Kp.: 37 °C/ 16<br>$n_D^{20}$ = 1,4268 |
| IX-6 | 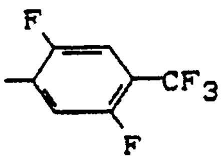 | F | Kp.: 104 °C/ 760<br>$n_D^{20}$ = 1,3862 |
| IX-7 | 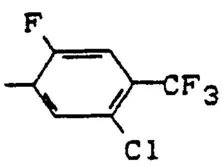 | F | Kp.: 130-140 °C / 760<br>$n_D^{20}$ = 1,4250 |

Anwendungsbeispiele

In den folgenden Anwendungsbeispielen wird die nachstehend aufgeführte Verbindung als Vergleichssubstanz herangezogen:

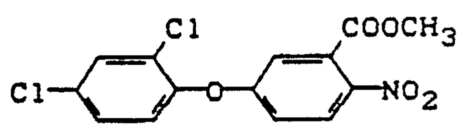

(A)

3-(2,4-Dichlor-phenoxy)-6-nitrobenzoesäure-methylester

(bekannt aus US-PS 36 52 645 und US-PS 3 776 715).

Beispiel A

Post-emergence-Test
Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit

der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welch eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß den Herstellungsbeispielen (1), (2), (3), (4) und (6) bei guter Selektivität in Mais erheblich stärkere Wirkung gegen Unkräuter, wie z. B. Amaranthus, Chenopodium, Ipomoea, Portulak und Solanum, als die Vergleichssubstanz (A).

## Beispiel B

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:
0 kein Austrocknen der Blätter, kein Blattfall
+ leichtes Austrocknen der Blätter, geringer Blattfall
+ + starkes Austrocknen der Blätter, starker Blattfall
+ + + sehr starkes Austrocknen der Blätter, sehr starker Blattfall

In diesem Test zeigen beispielsweise die Wirkstoffe gemäß den Herstellungsbeispielen (1) und (3) ein sehr starkes Austrocknen der Blätter und sehr starken Blattfall.

## Ansprüche

1. Halogensubstituierte Phenoxyphenylpropionsäure-Derivate der allgemeinen Formel (I)

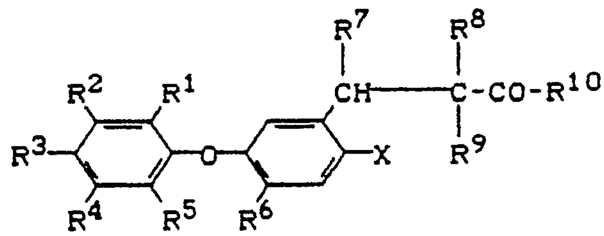

(I)

in welcher
X für Halogen steht,
$R^1$ für Wasserstoff, Halogen, Cyano oder Trifluormethyl steht,
$R^2$ für Waserstoff oder Halogen steht,
$R^3$ für Halogen, Cyano, Trifluormethyl, Trifluormethoxy oder Trifluormethylsulfonyl steht,
$R^4$, $R^5$, $R^6$, $R^7$, $R^8$ und $R^9$ unabhängig voneinander für Wasserstoff oder Halogen stehen,
$R^{10}$ für Halogen, Hydroxy, Amino, Alkylamino, Alkenylamino, Alkinylamino, Arylamino, Aralkylamino,

Alkoxycarbonyl-alkylamino, Cyanoamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonyl-alkylthio oder für die Gruppierung O-$R^{11}$ steht, worin

$R^{11}$ für einen gegebenenfalls durch Halogen substituierten Rest aus der Reihe Alkyl, Alkenyl, Alkinyl, Alkoxyalkyl, Alkylthioalkyl, Alkylsilfonylalkyl, Alkylsulfonylalkyl, Aryloxyalkyl, Trialkylsilylalkyl, Arylthioalkyl, Aralkoxyalkyl, Aralkylthioalkyl, Alkoxycarbonylalkyl, Alkylaminocarbonylalkyl, Aralkyl, Azolylalkyl oder für ein Ammonium-, Alkylammonium-, Alkalimetall- oder Erdalkalimetall-äquivalent

steht oder für die Gruppierung

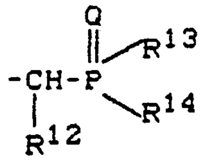

steht, worin

$R^{12}$ für Wasserstoff, Alkyl, Aryl, Furyl, Thienyl oder Pyridyl steht,

$R^{13}$ für Alkyl oder Alkoxy steht,

$R^{14}$ für Alkoxy steht und

Q für Sauerstoff oder Schwefel steht, oder

$R^{11}$ für die Gruppierung $-(CH_2)_n-R^{15}$ steht, worin

n für die Zahlen 0, 1 oder 2 steht und

$R^{15}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

2. Halogensubstituierte Phenoxyphenylpropionsäure-Derivate der Formel (I) gemäß Anspruch 1, in welcher

X für Fluor, Chlor oder Brom steht,

$R^1$ für Wasserstoff, Fluor, Chlor, Brom, Cyano oder Trifluormethyl steht,

$R^2$ für Wasserstoff, Fluor, Chlor oder Brom steht,

$R^3$ für Fluor, Chlor, Brom, Cyano, Trifluormethyl, Trifluormethoxy oder Trifluormethylsulfonyl steht,

$R^4$, $R^5$, $R^6$, $R^8$ und $R^9$ unabhängig voneinander für Wasserstoff, Fluor, Chlor oder Brom stehen,

$R^7$ für Wasserstoff, Chlor oder Brom steht,

$R^{10}$ für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxy-carbonyl-$C_1$-$C_2$-alkylamino, Cyanoamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung O-$R^{11}$ steht, worin

$R^{11}$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Tri methylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl oder für ein Ammonium-, ein $C_1$-$C_4$-Alkylammonium-, ein Natrium-, Kalium- oder Calcium-äquivalent steht, oder für die Gruppierung

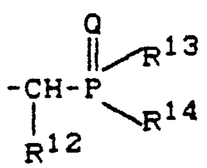

steht, worin

$R^{12}$ für Wasserstoff, $C_1$-$C_4$-Alkyl, Phenyl, Furyl, Thienyl oder Pyridyl steht,

$R^{13}$ für $C_1$-$C_4$-Alkyl oder $C_1$-$C_4$-Alkoxy steht,

$R^{14}$ für $C_1$-$C_4$-Alkoxy steht und

Q für Sauerstoff oder Schwefel steht, oder

$R^{11}$ für die Gruppierung $-(CH_2)_n-R^{15}$ steht, worin
n für die Zahlen 0, 1 oder 2 steht und
$R^{15}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

3. Halogensubstituierte Phenoxyphenylpropionsäure-Derivate der Formel (I) gemäß Anspruch 1, in welcher
X für Chlor oder Brom steht,
$R^1$ für Wasserstoff, Fluor oder Chlor steht,
$R^2$ für Wasserstoff, Fluor oder Chlor steht,
$R^3$ für Trifluormethyl oder Trifluormethylsulfonyl steht,
$R^4$ für Wasserstoff, Fluor oder Chlor steht,
$R^5$ für Wasserstoff, Fluor oder Chlor steht,
$R^6$ für Wasserstoff oder Chlor steht,
$R^7$ für Wasserstoff, Chlor oder Brom steht,
$R^8$ für Wasserstoff, Chlor oder Brom steht,
$R^9$ für Wasserstoff oder Chlor steht,
$R^{10}$ für Chlor, Hydroxy, Amino,$C_1$-$C_4$ Alkylamino, $C_1$-$C_4$-Alkoxy-carbonyl-$C_1$-$C_2$-alkylamino, Di-($C_1$-$C_3$-alkyl)-amino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung O-$R^{11}$ steht, worin
$R^{11}$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkyl-sulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl oder für ein Ammonium-, ein $C_1$-$C_3$-Alkylammonium-, ein Natrium- oder Kalium-äquivalent steht, oder für die Gruppierung

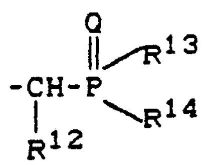

steht, worin
$R^{12}$ für Wasserstoff, Methyl, Phenyl, Furyl, Thienyl oder Pyridyl steht,
$R^{13}$ für $C_1$-$C_2$-Alkoxy steht,
$R^{14}$ für $C_1$-$C_2$-Alkoxy steht und
Q für Sauerstoff oder Schwefel steht, oder
$R^{11}$ für die Gruppierung $-(CH_2)_n-R^{15}$ steht, worin
n für die Zahlen 0, 1 oder 2 steht und
$R^{15}$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl und Dioxolanyl steht.

4. Verfahren zur Herstellung von halogensubstituierten Phenoxyphenylpropionsäure-Derivaten der allgemeinen Formel (I)

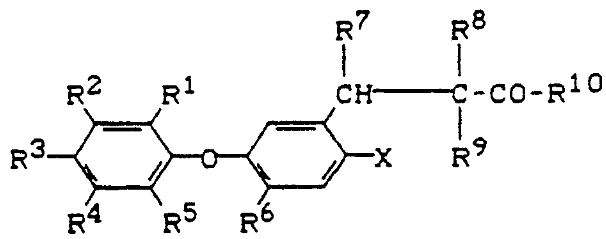

(I)

in welcher
X für Halogen steht,
$R^1$ für Wasserstoff, Halogen, Cyano oder Trifluormethyl steht,

$R^2$ für Wasserstoff oder Halogen steht,
$R^3$ für Halogen, Cyano, Trifluormethyl, Trifluormethoxy oder Trifluormethylsulfonyl steht,
$R^4$, $R^5$, $R^6$, $R^7$, $R^8$ und $R^9$ unabhängig voneinander für Wasserstoff oder Halogen stehen,
$R^{10}$ für Halogen, Hydroxy, Amino, Alkylamino, Alkenylamino, Alkinylamino, Arylamino, Aralkylamino, Alkoxycarbonyl-alkylamino, Cyanoamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonyl-alkylthio oder für die Gruppierung O-$R^{11}$ steht, worin
$R^{11}$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Trialkylsilylalkyl, Arythioalkyl, Aralkoxyalkyl, Aralkylthioalkyl, Alkoxycarbonylalkyl, Alkylaminocarbonylalkyl, Aralkyl, Azolylalkyl oder für ein Ammonium-, Alkylammonium-, Alkalimetall-oder Erdalkalimetall-äquivalent
steht oder für die Gruppierung

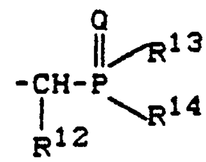

steht, worin
$R^{12}$ für Wasserstoff, Alkyl, Aryl, Furyl, Thienyl oder Pyridyl steht,
$R^{13}$ für Alkyl oder Alkoxy steht,
$R^{14}$ für Alkoxy steht und
Q für Sauerstoff oder Schwefel steht, oder
$R^{11}$ für die Gruppierung $-(CH_2)_n-R^{15}$ steht, worin
n für die Zahlen 0, 1 oder 2 steht und
$R^{15}$ für einen gegebenenfalls durch Halogen und/oder Alkyl substituierten heterocyclischen Rest aus der Reihe Furyl, Tetra hydrofuryl,Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht,
dadurch gekennzeichnet, daß man

(a) Phenoxyphenylaminoverbindungen der allgemeinen Formel (II)

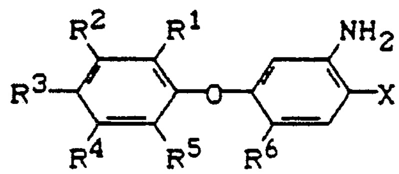

(II)

in welcher
$R^1$, $R^2$, $R^3$, $R^4$, $R^5$, $R^6$ und X die oben angegebene Bedeutung haben,
- oder Säureaddukte von Verbindungen der Formel (II) -
mit Natriumnitrit oder Kaliumnitrit und mit einem Hydrogenhalogenid (H-X') in Gegenwart von Wasser und gegebenenfalls in Gegenwart eines organischen Lösungsmittels umsetzt und die dabei gebildeten Diazoniumsalze der allgemeinen Formel (III)

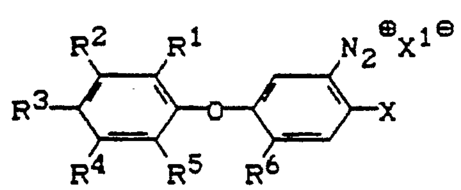

(III)

in welcher
$R^1$, $R^2$, $R^3$, $R^4$, $R^5$ und $R^6$ die oben angegebenen Bedeutungen haben und
X und X' gleich oder verschieden sind und für Halogen stehen,
mit Acrylsäure-Derivaten der allgemeinen Formel (IV)

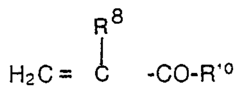 (IV)

in welcher
$R^8$ und $R^{10}$ die oben angegebenen Bedeutungen haben,
in Gegenwart von Hydrogenhalogeniden ($HX'$), gegebenenfalls in Gegenwart von Katalysatoren und gegebenenfalls in Gegenwart von Wasser und dem gegebenenfalls bei der Erzeugung der Verbindung der Formel (III) verwendeten organischen Lösungsmittel umsetzt oder daß man

(b) für den Fall, daß $R^7$, $R^8$ und $R^9$ für Wasserstoff stehen und die Reste $R^1$ bis $R^6$ sowie $R^{10}$ und X die oben angegebenen Bedeutungen haben, Verbindungen der Formel (I), in welcher $R^7$, $R^8$ und/oder $R^9$ für Halogen stehen und die Reste $R^1$ bis $R^6$ sowie $R^{10}$ und X die oben angegebenen Bedeutungen haben, mit Wasserstoff in Gegenwart eines Hydrierungskatalysators und in Gegenwart eines Verdünnungsmittels umsetzt, oder daß man

(c) für den Fall, daß $R^7$, $R^8$ und $R^9$ für Wasserstoff stehen und die Reste $R^1$ bis $R^6$ sowie $R^{10}$ und X die oben angegebenen Bedeutungen haben, Verbindungen der Formel (V)

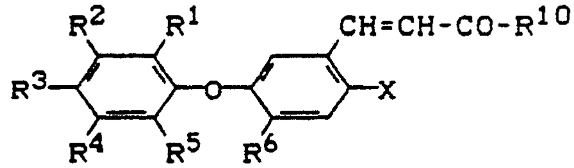 (V)

in welcher
$R^1$, $R^2$, $R^3$, $R^4$, $R^5$, $R^6$, $R^{10}$ und X die oben angegebenen Bedeutungen haben,
mit Wasserstoff in Gegenwart eines Hydrierungskatalysators und in Gegenwart eines Verdünnungsmittels umsetzt, oder daß man

(d) für den Fall, daß $R^7$ für Chlor oder Brom steht und die Reste $R^1$ bis $R^6$ sowie $R^8$ bis $R^{10}$ und X die oben angegebenen Bedeutungen haben, Verbindungen der Formel (I), in welcher $R^7$ für Wasserstoff steht und die Reste $R^1$ bis $R^6$ sowie $R^8$ bis $R^{10}$ und X die oben angegebenen Bedeutungen haben, mit N-Chlor- bzw. N-Brom-succinimid in Gegenwart von 2,2′-Azoisobuttersäure-dinitril (Azoisobutyronitril) und von Tetrachlormethan umsetzt, oder daß man

(e) für den Fall, daß $R^{10}$ für Hydroxy steht und die Reste $R^1$ bis $R^9$ und X die oben angegebenen Bedeutungen haben, Verbindungen der Formel (VI)

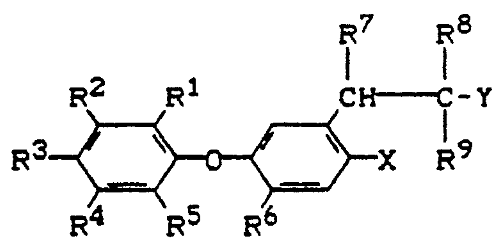 (VI)

in welcher
$R^1$, $R^2$, $R^3$, $R^4$, $R^5$, $R^6$, $R^7$, $R^8$, $R^9$ und X die oben angegebenen Bedeutungen haben und
Y für Cyano, Methoxycarbonyl oder Ethoxycarbonyl steht,
mit Wasser, gegebenenfalls in Gegenwart eines Verseifungshilfsmittels und gegebenenfalls in Gegenwart eines organischen Lösungsmittels umsetzt, oder daß man

(f) für den Fall, daß $R^{10}$ für Halogen steht und die Reste $R^1$ bis $R^9$ und X die oben angegebenen Bedeutungen haben, Verbindungen der Formel (I), in welcher $R^{10}$ für Hydroxy steht und die Reste $R^1$ bis $R^9$ und X die oben angegebenen Bedeutungen haben,
mit einem Halogenierungsmittel, gegebenenfalls in Gegenwart eines Katalysators und gegebenenfalls in Gegenwart eines organischen Lösungsmittels, umsetzt, oder daß man

(g) für den Fall, daß $R^{10}$ mit Ausnahme von Halogen die oben angegebene Bedeutung hat und die Reste $R^1$ bis $R^9$ und X die oben angegebenen Bedeutungen haben, Verbindungen der Formel (I), in welcher $R^{10}$ für Halogen steht und die Reste $R^1$ bis $R^9$ und X die oben angegebenen Bedeutungen haben mit Verbindungen der Formel (VII)

$H\text{-}R^{10}$ (VII)

in welcher
$R^{10}$ mit Ausnahme von Halogen die oben angegebene Bedeutung hat,
gegebenenfalls in Gegenwart eines Säureakzeptors und gegebenenfalls in Gegenwart eines Verdünnungsmittels umsetzt, oder daß man

(h) für den Fall, daß X für Chlor oder Brom steht und die Reste $R^1$ bis $R^{10}$ die oben angegebenen Bedeutungen haben, Verbindungen der Formel (VIII)

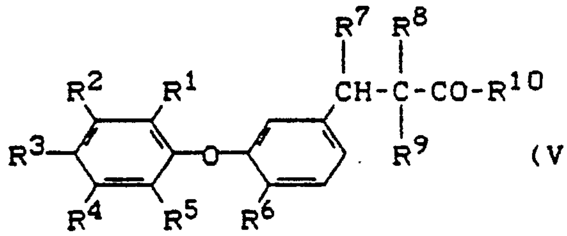
(VIII)

in welcher
$R^1$, $R^2$, $R^3$, $R^4$, $R^5$, $R^6$, $R^7$, $R^8$, $R^9$ und $R^{10}$ die oben angegebenen Bedeutungen haben,
mit Chlor oder Brom, gegebenenfalls in Gegenwart eines Verdünnungsmittels umsetzt.

5. Herbizide und pflanzenwuchsregulierende Mittel, gekennzeichnet durch einen Gehalt an mindestens einem halogensubstituierten Phenoxyphenylpropionsäure-Derivat der Formel (I) gemäß den Ansprüchen 1 bis 4.

6. Verfahren zur Bekämpfung von Unkräutern, dadurch gekennzeichnet, daß man halogensubstituierte Phenoxyphenylpropionsäure-Derivate der Formel (I) gemäß den Ansprüchen 1 bis 4 auf die Unkräuter und/oder ihren Lebensraum einwirken läßt.

7. Verwendung von halogensubstituierten Phenoxyphenylpropionsäure-Derivaten der Formel (I) gemäß den Ansprüchen 1 bis 4 zur Bekämpfung von Unkräutern und/oder als Pflanzenwachstumsregulatoren.

8. Verfahren zur Herstellung von herbiziden und/oder pflanzenwuchsregulierenden Mitteln, dadurch gekennzeichnet, daß man halogensubstituierte Phenoxyphenylpropionsäure-Derivate der Formel (I) gemäß den Ansprüchen 1 bis 4 mit Streckmitteln und/oder oberflächenaktiven Substanzen vermischt.

9. Phenoxyphenylamino-Verbindungen der Formel (II)

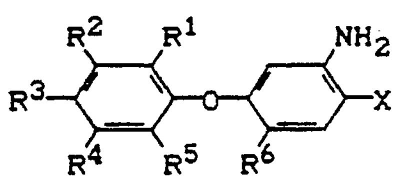
(II)

in welcher
X für Halogen steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht und
$R^4$, $R^5$ und $R^6$ unabhängig voneinander für Wasserstoff oder Halogen stehen.

10. Verfahren zur Herstellung von Phenoxyphenylamino-Verbindungen der Formel (II)

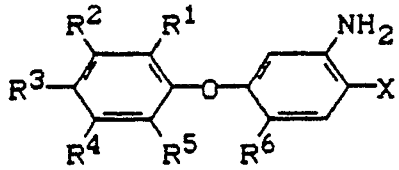

(II)

in welcher
X für Halogen steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht und
$R^4$, $R^5$ und $R^6$ unabhängig voneinander für Wasserstoff oder Halogen stehen,
dadurch gekennzeichnet, daß man Halogen-benzol-Derivate der allgemeinen Formel (IX)

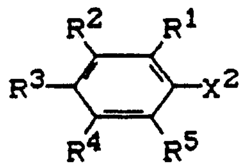

(IX)

in welcher
$R^1$, $R^2$, $R^3$, $R^4$ und $R^5$ die oben angegebenen Bedeutungen haben und
$X^2$ für Fluor oder Chlor steht,
mit Aminophenolen der allgemeinen Formel (X)

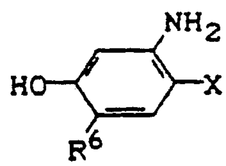

(X)

in welcher
$R^6$ und X die oben angegebenen Bedeutungen haben,
in Gegenwart eines Säureakzeptors und in Gegenwart eines Verdünnungsmittels umsetzt.

11. Verbindungen der Formel (V)

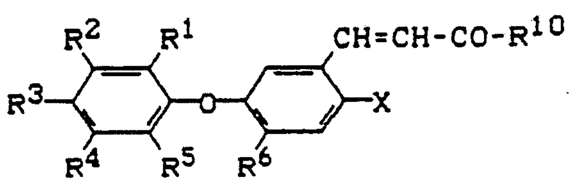

(V)

in welcher
X für Halogen steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$, $R^5$ und $R^6$ unabhängig voneinander für Wasserstoff oder Halogen stehen und
$R^{10}$ für Halogen, Hydroxy, Amino, Alkylamino, Alkenylamino, Alkinylamino, Arylamino, Aralkylamino, Alkoxycarbonyl-alkylamino, Cyanoamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonyl-alkylthio oder für die Gruppierung O-$R^{11}$ steht, worin
$R^{11}$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Trialkylsilylalkyl, Arylthioalkyl,

Aralkoxyalkyl, Aralkylthioalkyl, Alkoxycarbonylalkyl, Alkylaminocarbonylalkyl, Aralkyl, Azolylalkyl oder für ein Ammonium-, Alkylammonium-, Alkalimetall- oder Erdalkalimetall-äquivalent steht oder für die Gruppierung

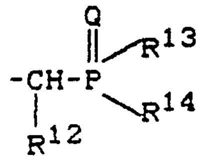

steht, worin
$R^{12}$ für Wasserstoff, Alkyl, Aryl, Furyl, Thienyl oder Pyridyl steht,
$R^{13}$ für Alkyl oder Alkoxy steht,
$R^{14}$ für Alkoxy steht und
Q für Sauerstoff oder Schwefel steht, oder
$R^{11}$ für die Gruppierung $-(CH_2)_n-R^{15}$ steht, worin
n für die Zahlen 0, 1 oder 2 steht und
$R^{15}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

12. Verbindungen der Formel (VI)

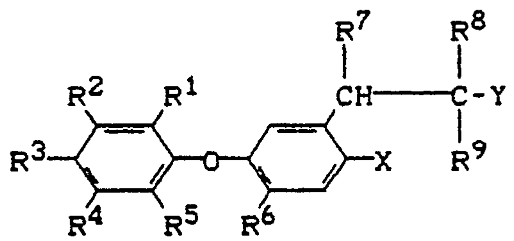

(VI)

in welcher
X für Halogen steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$, $R^5$, $R^6$, $R^7$, $R^8$ und $R^9$ unabhängig voneinander für Wasserstoff oder Halogen stehen und
Y für Cyano, Methoxycarbonyl oder Ethoxycarbonyl steht.